# EUROPEAN PATENT APPLICATION

(11) **EP 1 138 658 A1**
(43) Date of publication of application: **04.10.2001**
(21) Application number: 00963083.1
(22) Date of filing: 03.10.2000
(51) Int. Cl.: C07B 61/00, G06F 17/30, G06F 17/50

(54) **MOLECULE STEREOCHEMICAL CODING METHOD**

(30) Priority: 08.10.1999 JP 28869199
(71) Applicant: Riken, Wako-shi, Saitama 351-0198 (JP); Japan Science and Technology Corporation, Kawaguchi-shi, Saitama 332-0012 (JP)
(72) Inventor: KOSHINO, Hiroyuki, Wako-shi, Saitama 351-0198 (JP); SATOH, Hiroko, Wako-shi, Saitama 351-0114 (JP); NAKATA, Tadashi, Wako-shi, Saitama 351-0198 (JP)
(74) Representative: Liesegang, Eva
(86) International application number: JP0006887
(87) International publication number: WO0127052

(57) **Abstract**

There is provided a molecular coding method capable of being applied to, e.g., the prediction of the NMR chemical shift, and computer-readably and canonically coding information on a stereostructural environment, such as conformation and configuration, of a molecule.

In a molecular stereochemical coding method for taking a stereochemistry about each of a plurality of atoms constituting a molecule to code the molecule, atoms are sequentially assigned to hierarchies, and a predetermined precedence rule for placing a plurality of atoms belonging the same hierarchy in the order.

Then, molecular trees are formed from a lower hierarchy to a higher hierarchy so as to express the bonding relationship between the plurality of atoms. Then, a dihedral angle is derived with respect to a group comprising an atom in the (n+3)-th hierarchy, which is to be noted, an atom in the (n+2)-th hierarchy, an atom in the (n+1)-th hierarchy and an atom in the n-th hierarchy. In accordance with the magnitude of the dihedral angle, the dihedral angle is replaced with an angular symbol which is defined in accordance with a predetermined angle dividing rule, and a symbol is given to the noticed atom in the (n+3)-th hierarchy. Then, a predetermined linear rule for expressing the molecular tree by a row of characters is set, and a linear notation is carried out so as to correspond to the molecular tree, to prepare codes concerning a stereochemistry indicative of conformation and configuration of the molecule with respect to the start atom.

## Description

### TECHNICAL FIELD

The present invention relates generally to a molecular stereochemical coding method. More specifically, the invention relates to a molecular stereochemical coding method for taking a stereochemistry about each of a plurality of atoms constituting a molecule to code the molecule.

### BACKGROUND ART

As methods for describing stereochemical information of a molecular structure so as to be capable of writing and reading the stereochemical information in and out of a computer, there are a method for describing the positions of atoms in a three-dimensional coordinate system such as the Cartesian coordinate system or internal coordinate system, and a method for mathematically describing the topological relationship between atoms for the purpose of unique nomenclature of a molecule including stereochemistry.

Since the former method for describing the positions of atoms in the three-dimensional coordinate system can not uniquely express them, it is difficult or complicated to compare the similarity and/or homology of molecules. The latter method for mathematically describing the topological relationship between atoms can express the absolute configuration (R, S) of asymmetric carbon and so forth. However, this method can not describe relative steric environment between optional atoms, so that it is not possible to express information on conformation and so forth in the present circumstances.

For example, when it is intended to predict the NMR chemical shift from the existing data base, stereochemical information suitable therefor has not been canonically coded. For that reason, only information on planar structure has been used in the present circumstances. The prediction of the chemical shift based on the data base taking account of stereochemistry has not been realized even in any conventional chemical shift predicting computer systems.

In addition, there are not any notation techniques capable of easily utilizing information on a difference in stereostructural environment, such as conformation and configuration, between a plurality of molecules, as computer readable data.

### DISCLOSURE OF THE INVENTION

It is therefore an object of the present invention to solve the above described problems of the prior art and to provide a molecular coding method for computer-readably and canonically coding information on stereostructural environment, such as conformation and configuration, of a molecule.

In order to the above described object, according to a first aspect of the present invention, there is provided a molecular stereochemical coding method for taking a stereochemistry about each of a plurality of atoms constituting a molecule to code the molecule, the method comprising: a hierarchy classifying step of assigning a start atom, which is to be noticed, to a zero-th hierarchy serving as the lowest hierarchy, assigning an atom, which is combined with the start atom on a higher hierarchy side, to a first hierarchy, assigning an atom, which is combined with the atom assigned to the first hierarchy, to a second hierarchy, and similarly, sequentially assigning atoms to hierarchies until the final hierarchy which is set so as to be specially requested; a molecular tree forming step of setting a predetermined precedence rule for placing a plurality of atoms, which belong to the same hierarchy, in the order, and placing the atoms, which belong the same hierarchy, in the order every hierarchy in accordance with the precedence rule, to form a molecular tree every the start atom from a lower hierarchy to a higher hierarchy so as to express a bonding relationship between the plurality of atoms; a coding step of noticing one of atoms, which are assigned to the (n+3)-th hierarchy, with respect to each of integers n assuming that n is an integer of 0 or more, in the molecular tree, deriving a dihedral angle between a plane, which is formed by an atom in the (n+3)-th hierarchy, an atom in the (n+2)-th hierarchy and an atom in the (n+1)-th hierarchy, and a plane, which is formed by the atom in the (n+2)-th hierarchy, the atom in the (n+1)-th hierarchy and an atom in the n-th hierarchy, with respect to a group comprising four atoms which consists of the noticed atom in the (n+3)-th hierarchy, the atom in the (n+2)-th hierarchy which is combined with the atom in the (n+3)-th hierarchy, the atom in the (n+1)-th hierarchy which is combined with the atom in the (n+2)-th hierarchy, and the atom in the n-th hierarchy which is combined with the atom in the (n+1)-th hierarchy, replacing the derived dihedral angle into an angular symbol, which is defined in accordance with a predetermined angle dividing rule, in accordance with the magnitude of the dihedral angle, giving the replaced angular symbol to the noticed atom in the (n+3)-th hierarchy, and similarly, giving angular symbols in accordance with the magnitudes of the dihedral angles with respect to other atoms to be noticed; and setting a predetermined linear notation rule for expressing the molecular tree by a row of characters, carrying out the linear notation of a set of the plurality of angular symbols in accordance with the predetermined linear notation rule so as to correspond to the molecular tree, preparing a conformation code indicative of a conformation of the molecule with respect to the start atom, and similarly, preparing conformation codes with respect to other start atoms.

According to the first aspect of the present invention, since the four atoms are sequentially assigned from the atom belonging to the (n+3)-th hierarchy, which is noticed in the molecular tree, to the atom belonging to the n-th hierarchy to be set as a group, the group comprising the four atoms is uniquely determined, so that the dihedral angle in this group is uniquely derived. Since the linear notation of the molecular tree expressed by the angular symbols is carried out in the predetermined linear notation rule to form the conformation codes, stereochemical information can be computer-readably coded. By comparing the conformation codes between compared molecules, the difference in conformation can be determined.

According to a second aspect of the present invention, the molecular stereochemical coding method according the first aspect of the present invention further comprises a configuration code preparing step of preparing a configuration code indicative of a configuration of the molecule every the start atom, wherein the configuration code preparing step causes an atom, which is to be noticed in the n-th hierarchy, to be positioned at a reference position in the angle dividing rule, integrally rotates the atoms belonging to the (n+3)-th hierarchy about a bonding axis, which connects the atom in the (n+1)-th hierarchy to the atom in the (n+2)-th hierarchy, so that an atom, which has a predetermined precedence in accordance with the precedence rule among the atoms belonging to the (n+3)-th hierarchy, is positioned at a predetermined angular position with respect to the reference position, gives an angular symbol according to the angle dividing rule to each of the atoms belonging to the (n+3)-th hierarchy, in accordance with an angular position after rotation with respect to the reference position, carries out the linear notation of a set of the plurality of angular symbols in accordance with the predetermined linear notation rule so as to correspond to the molecular tree, and prepares a configuration code every the start atom.

According to the second aspect of the present invention, since it is possible to obtain the configuration codes indicative of the linearly expressed molecular configuration in addition to the conformation codes, stereochemistry information including conformation and configuration can be computer-readably coded.

According to a third aspect of the present invention, the molecular stereochemical coding method according to the second aspect of the present invention further comprises a planar structure code preparing step of preparing a planar structure code indicative of a planar structure of the molecule every the start atom, wherein the planar structure code preparing step expresses the molecular tree by a set of planar structure symbols which planar-structurally express a bonding relationship between the plurality of atoms, carries out the linear notation of the set of planar structure symbols in accordance with the predetermined linear notation rule so as to correspond to the molecular tree, prepares a planar structure code indicative of the planar structure of the molecule with respect to the start atom, and similarly, prepares planar structure codes with respect to other start atoms.

According to the third aspect of the present invention, since it is possible to obtain the planar structure codes indicative of the linearly expressed molecular planar structure in addition to the conformation codes and configuration codes, it is possible to easily carry out the total comparison between molecules, the planar structures, conformations and configurations of which are compared.

In the molecular stereochemical coding method according to the third aspect of the present invention, the conformation codes, the configuration codes and the planar structure codes may be expressed in parallel with respect to the start atoms. Thus, it is possible to easily compare the conformation codes and the configuration codes while referring to the planar structure codes indicative of the planar structure of the molecule.

In the molecular stereochemical coding method according to the third aspect of the present invention, when it is impossible to rotate the atoms belonging to the (n+3)-th hierarchy about the bonding axis connecting the atom belonging to the (n+1)-th hierarchy to the atom belonging to the (n+2)-th hierarchy, the angular symbols given at the coding step may be adopted as they are, and the linear notation of a set of the plurality of angular symbols may be carried out in accordance with the predetermined linear notation rule so as to correspond to the molecular tree, to prepare the configuration codes for every the start atom. Thus, even if it is impossible to rotate the atoms belonging to the (n+3)-th hierarchy about the bonding axis connecting the atom belonging to the (n+1)-th hierarchy to the atom belonging to the (n+2)-th hierarchy, it is possible to form the configuration codes.

In the molecular stereochemical coding method according to the third aspect of the present invention, the planar structural codes may be CANOST linear notations. Thus, it is possible to utilize the existing technique.

In the molecular stereochemical coding method according to the first aspect of the present invention, the precedence rule may be a CANOST code precedence rule. Thus, it is possible to utilize the existing technique.

In the molecular stereochemical coding method according to the first aspect of the present invention, the predetermined linear notation rule may be a CANOST code linear notation rule. Thus, it is possible to utilize the existing technique.

In the molecular stereochemical coding method according to the first aspect of the present invention, at the molecular tree forming step, a group of atoms having a low degree of notice in the identification of stereochemistry may be replaced with predetermined symbols to be masked. Thus, it is possible to ignore a difference, which is not based on the essential structural difference, to examine a difference which is based on a considerable structural difference.

In the molecular stereochemical coding method according to the first aspect of the present invention, the predetermined angle dividing rule may divide an angle of 360 degrees into a predetermined number of clock-dial-like angular ranges, and the divided angular ranges may be reflected in the level of abundance to be unequally divided. Thus, it is possible to enhance the applicability and reliability of the conformation codes.

In the molecular stereochemical coding method according to the first aspect of the present invention, at the conformation code preparing step, the conformation codes may be prepared with respect to at least two of the start atoms, the hierarchy numbers of which are spaced from each other by three hierarchies or more.

According to a fourth aspect of the present invention, there is provided a computer readable recording media in which a program for executing the molecular stereochemical coding method according to the first aspect of the present invention has been recorded. Thus, the molecular stereochemical coding method according to the first aspect of the present invention can be easily executed by means of a computer.

### BRIEF DESCRIPTION OF THE DRAWINGS

FIG. 1 is a diagram showing the procedure for forming a molecular tree and a CANOST linear notation (a planar structure code) using a-Glc-4C as a model molecular, wherein (a) shows the conformation of a-Glc-4C, (b) shows a first molecular tree in a hierarchy classifying process, (c) shows a second molecular tree, (d) shows a third molecular tree and (e) shows a CANOST linear notation (a planar structure code);
FIG. 2 is an enlarged view of FIG. 1(a);
FIG. 3 is a diagram for explaining the procedure for deriving a dihedral angle;
FIG. 4 is a table showing a CANOST code list;
FIG. 5 is a diagram showing a molecular tree and a CANOST linear notation which are prepared using a-Glc-4C-chain as a model molecule;
FIG. 6 is a diagram showing an angle dividing rule for coding a dihedral angle in accordance with its magnitude;
FIG. 7 is a diagram showing (a) a staggered type conformation and (b) an eclipsed type conformation;
FIG. 8 is a diagram showing a conformation code preparing step by a Newman projection drawing;
FIG. 9 is a diagram for explaining the procedure for deriving configuration codes;
FIG. 10 is a diagram showing the procedure for deriving conformation codes with respect to atoms of sp² type;
FIG. 11 is a diagram showing the procedure for deriving configuration codes with respect to atoms of sp² type;
FIG. 12 is a diagram showing (a) a planer structural formula of a molecule shown in FIGS. 15 and 16 and (b) a planar structural formula of a molecule shown in FIG. 18;
FIG. 13 is a diagram showing (a) an example of a configuration of a molecule shown in FIGS. 15 and 16 and (b) an example of a configuration of a molecule shown in FIG. 18;
FIG. 14 is a diagram showing the comparison of a conformation of a-Glc-4C with a conformation of a-Glc-1C;
FIG. 15 is a diagram showing a stereostructural formula of a molecule;
FIG. 16 is a diagram showing a stereostructural formula of a molecule;
FIG. 17 is a diagram showing a stereostructural formula of a molecule;
FIG. 18 is a diagram showing a stereostructural formula of a molecule;
FIG. 19 is a diagram showing a stereostructural formula of a molecule;
FIG. 20 is a diagram showing a planar structure code (CANOST), conformation code (Stereo) and configuration code (Configuration) of a-Glc-4C;
FIG. 21 is a diagram showing conformation codes (Stereo) of a-Glc-4C when no masking is carried out and when a masking is carried out;
FIG. 22 is a diagram showing the compared results of conformation codes (Stereo) of b-Glc-4C with those of a-Glc-4C when no masking is carried out and when a masking is carried out;
FIG. 23 is a diagram showing the comparison of conformation codes (Stereo) of only two nodes 6 and 7 which are selected as start atoms with respect to various molecules, the stereostructural formulae of which are shown in FIGS. 15 and 16;
FIG. 24 is a diagram showing the comparison of conformation codes (Stereo) of only two nodes 6 and 7 which are selected as start atoms with respect to various molecules, the stereostructural formulae of which are shown in FIGS. 15 and 16;
FIG. 25 is a diagram showing the comparison of configuration codes (Configuration) of only two nodes 6 and 7 which are selected as start atoms with respect to various molecules, the stereostructural formulae of which are shown in FIGS. 15 and 16;
FIG. 26 is a diagram showing the comparison of conformation codes (Stereo), for which no masking is carried out, with respect to various molecules, the stereostructural formulae of which are shown in FIG. 18;
FIG. 27 is a diagram showing the comparison of conformation codes (Stereo), for which a masking is carried out, with respect to various molecules, the stereostructural formulae of which are shown in FIG. 18;
FIG. 28 is a diagram showing the comparison of configuration codes (Configuration) with respect to various molecules, the stereostructural formulae of which are shown in FIG. 18;
FIG. 29 is a diagram showing the comparison of conformation codes (Stereo) and configuration codes (Configuration) of Br atom and Cl atom, which are only two atoms selected as start atoms with respect to various molecules, the chemical formulae of which are shown in FIG. 19; and
FIG. 30 is a flow chart showing an example of the use of conformation codes (Stereo) and configuration codes (Configuration).

### BEST MODE FOR CARRYING OUT THE INVENTION

Referring to the accompanying drawings, the preferred embodiments of a molecular coding method according to the present invention will be described below.

First, a model molecule adopted to explain a molecular stereochemical coding method according to the present invention will be described.

Sugars were used as model molecules since sugars can be expressed in the form of cyclic molecules and chain molecules and have various conformations and many stereoisomers.

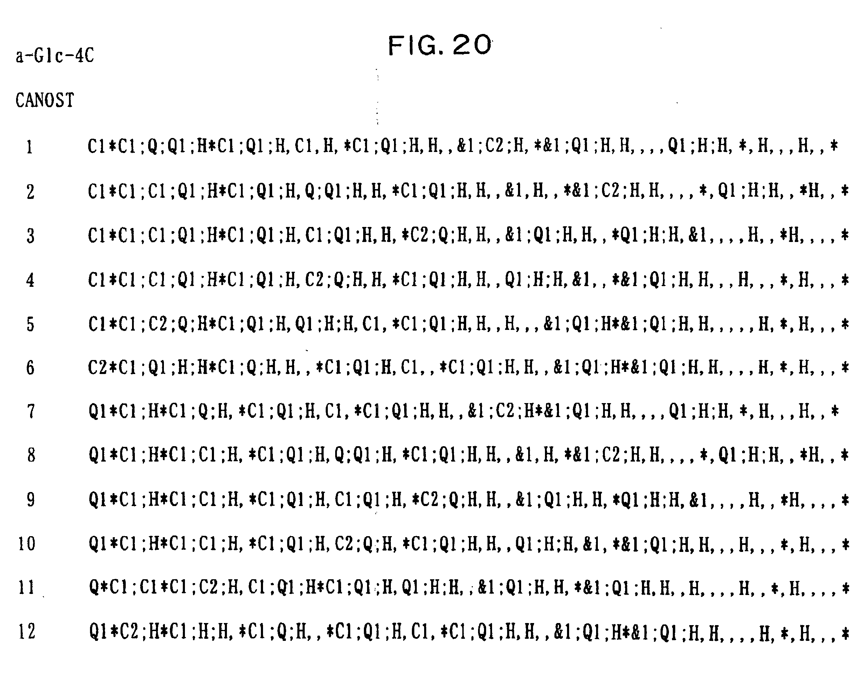

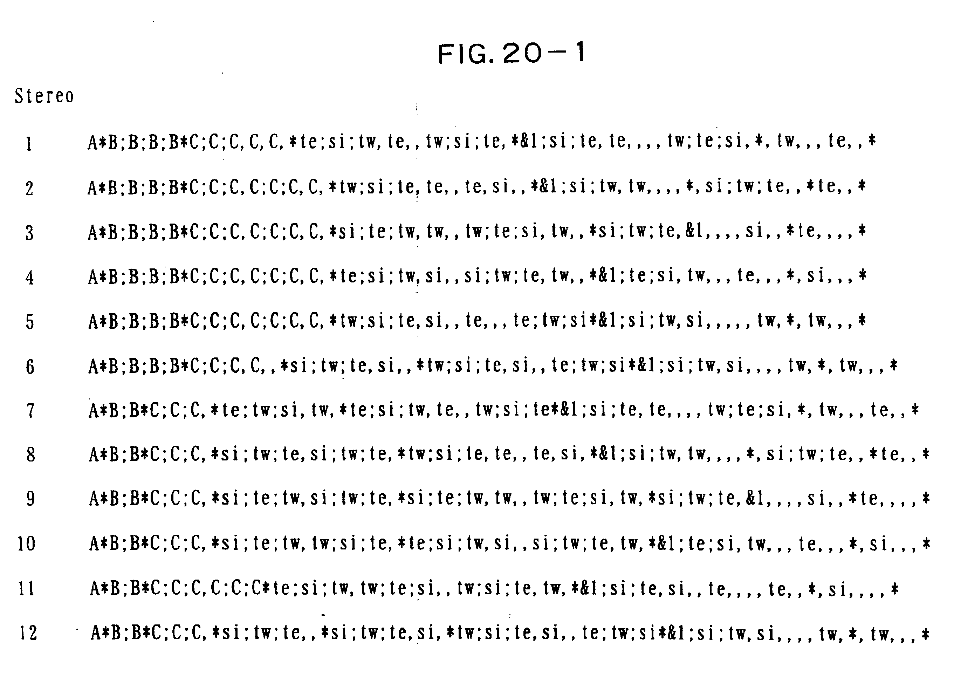

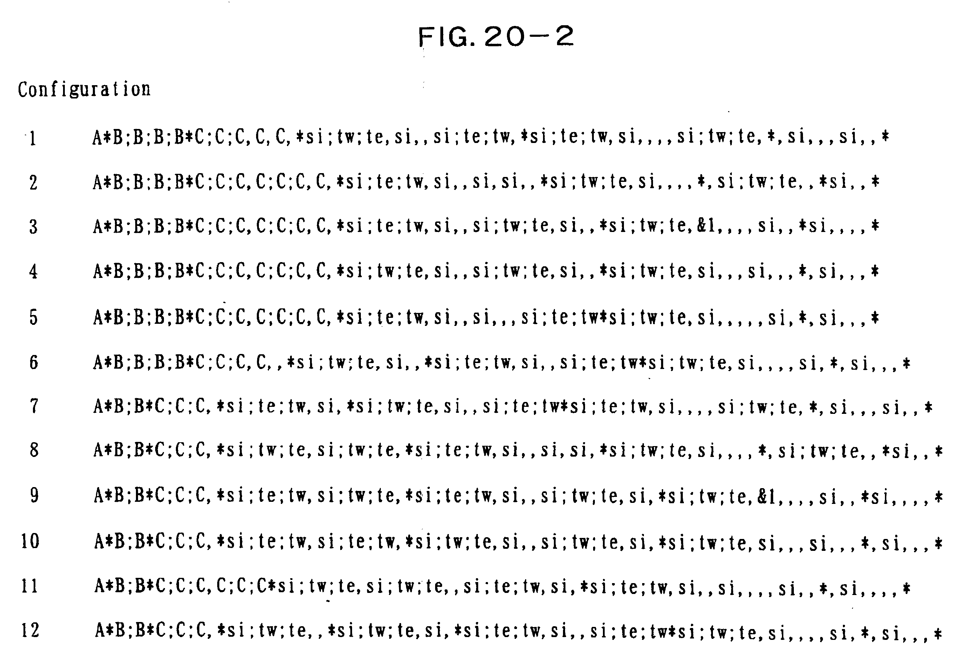

Specifically, D-glucose (abbreviation: Glc), D-galactose (abbreviation: Gal) which is an isomer at the 4-position, D-mannose (abbreviation: Man) which is an isomer at the 2-position, and so forth were used as model molecules. With respect to stereochemistry at the 1-position, one having "a-", such as a-Glc, shows a configuration of α-Glc, and one having "b-" shows a configuration of β-Glc. Similarly in FIG. 20 and so forth, one having "a-", such as a-Glc, shows a coordination of α-Glc, and one having "b-" shows a coordination of β-Glc.

In addition, 1C and 4C show a conformation wherein a carbon atom at the 1-position or 4-position is arranged at the apex of a chair form, 14B shows a conformation wherein carbon atoms at the 1-position and 4-position are arranged at both ends of a boat form, and 14TW shows a conformation of a twist-boat form derived from the conformation of 14B.

These sugars also take chain forms wherein a reducing terminal at the 1-position is aldehyde (-CHO). Among those taking chain forms, one holding a conformation of the original cyclic structure is expressed so that a cyclic conformation, such as 4C-chain, is also described. In addition, one having "-linear" at the end, such as 4C-chain-linear, shows one of stable conformations wherein a carbon chain is linear. Moreover, a-Glc-1C-chain-H-bond shows a conformation capable of forming a hydrogen bond between a hydroxyl group at the 3-position and a carbonyl group at the 1-position.

As double bond models, four kinds of geometrical isomers of 1-bromo-4-chlorobutadiene were used. The conformation is a planar conformation wherein the central single bond and the double bond are arranged on the same plane.

Referring to the accompanying drawings, a method for coding the stereochemistry of a molecule will be specifically described below.

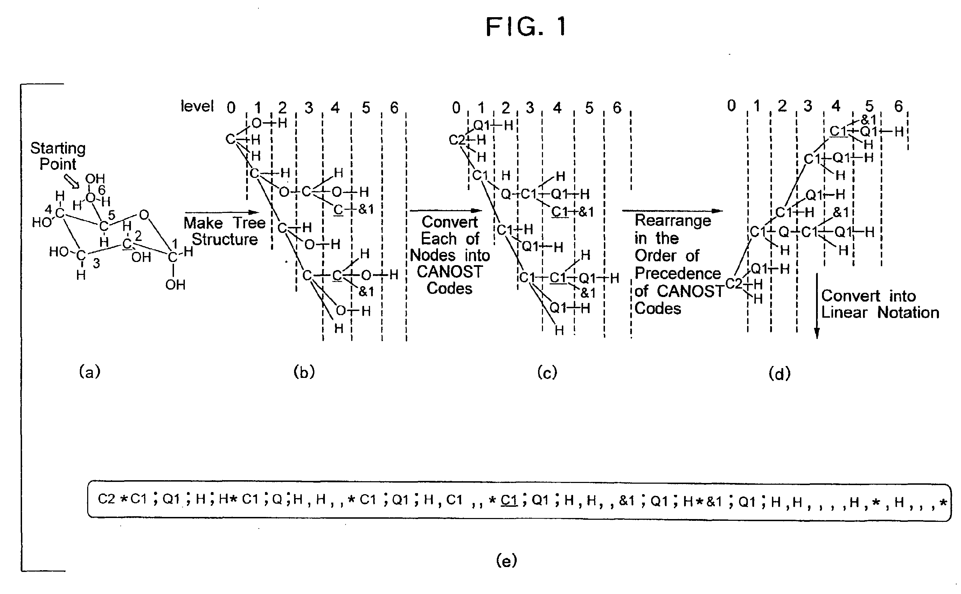

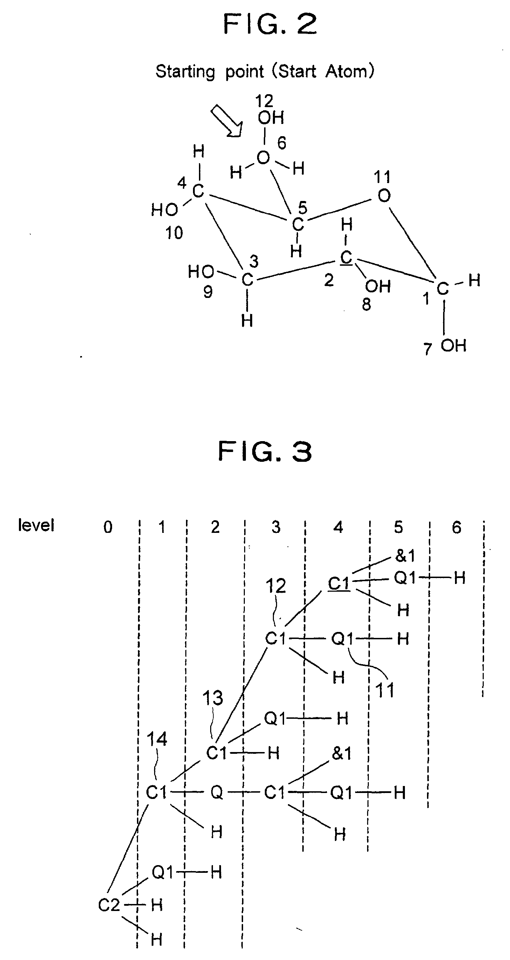

FIG. 1 shows the procedure for forming a molecular tree and a CANOST linear notation (a planar structure) using a-Glc-4C as a model molecule. FIG. 1(a) shows a conformation of a-Glc-4C, and the same conformation is enlarged to be shown in FIG. 2.

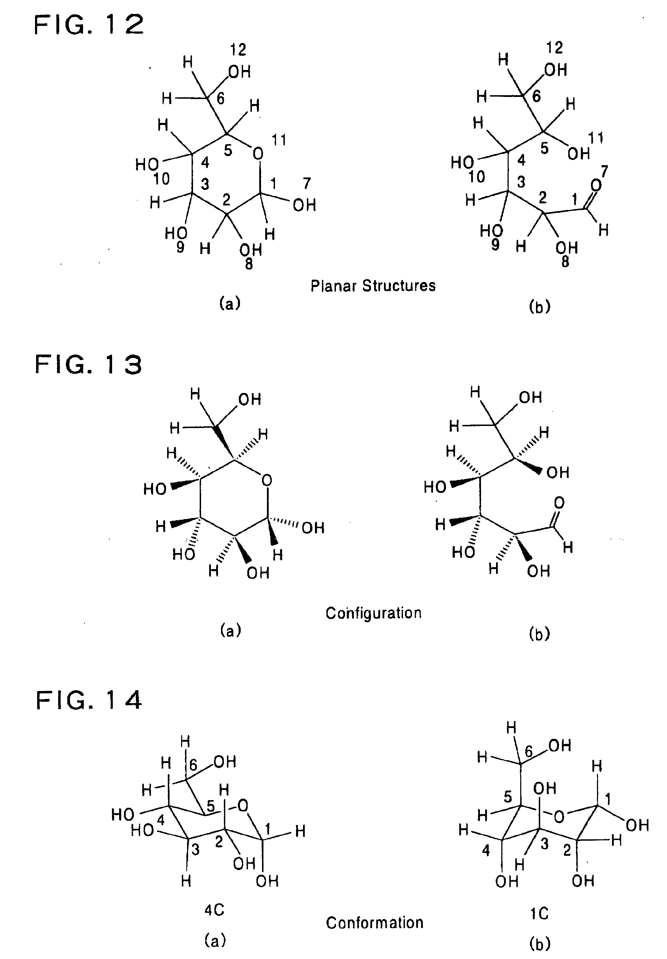

With respect to a-Glc-4c, as compared with a-Glc-4C-chain which will be described later, the planar structure of a-Glc-4C is shown in FIG. 12(a), and the configuration of a-Glc-4C is shown in FIG. 13(a).

Node numbers 1 through 6 correspond to carbon atoms C1 through C-6, and node numbers 7 through 12 correspond to O-1 through O-6.

The carbon atom of the node 6 is herein noticed as an example, and the carbon atom of the node 6 is set as a start atom. Subsequent procedures are similarly carried out every noticed start atom.

First, a hierarchy classifying process according to the present invention will be described below.

As shown in FIG. 1(b), a start atom is classified as level 0, and four atoms O, H, H and C combined with the start atom on the higher hierarchy side are classified as level 1. In addition, the bonds of the four atoms to the start atom are expressed, and the four atoms O, H, H and C are vertically arranged. Similarly, atoms combined with the atoms of level 1 on the higher hierarchy side are classified as level 2, and similarly, atoms are sequentially classified into hierarchies until the final hierarchy which is set so as to be specially requested. Thus, a first molecular tree shown in FIG. 1(b) is formed at the hierarchy classifying step.

The final hierarchy which is set so as to be specially requested may be set in accordance with the degree of similarity between molecules serving as objects to be compared, in view of the need for strictly deriving the degree. As the final hierarchy is set to be a deeper or higher hierarchy from the start atom, it is possible to strictly determine the degree of similarity.

A molecular tree forming step according to the present invention will be described below.

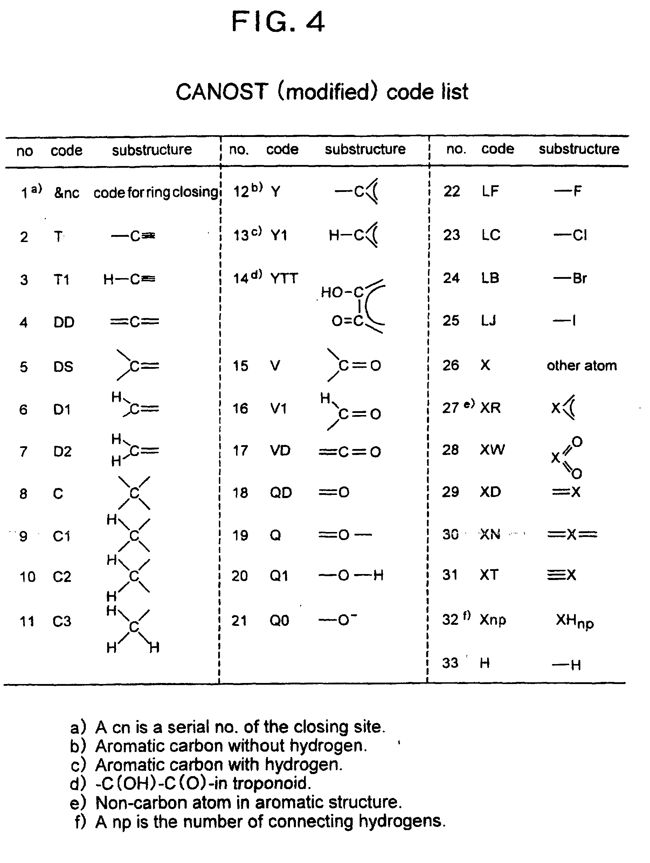

Atomic symbols in the first molecular tree shown in FIG. 1(b) are converted into CANOST codes in accordance with a CANOST code list (CANOST CODE LIST) shown in FIG. 4. As a result, as shown in FIG. 1(c), a second molecular tree is formed. In the CANOST code list shown in FIG. 4, a precedence is defined in order of figures included in column "no.", and partial structures included in column "substructure" are expressed by CANOST codes included in column "code".

Then, with respect to a second molecular tree shown in FIG. 1(c), a predetermined precedence rule for placing a plurality of atoms, which belong to the same hierarchy, in the order is set. A precedence rule described in the CANOST code list shown in FIG. 4 is herein adopted as the predetermined precedence rule. When the CANOST code precedence rule is adopted, lower hierarchies are preferentially sequentially adopted to arrange CANOST codes every hierarchy.

The CANOST codes are rearranged in accordance with the CANOST code precedence rule to form a third molecular tree shown in FIG. 1(d). In the third molecular tree, the plurality of atoms belonging to the same hierarchy are arranged so that the precedence decreases from top to bottom. The third molecular tree enlarged in FIG. 3 corresponds to a molecular tree according to the present invention.

A predetermined linear notation rule for expressing a molecular tree by a row of characters will be described below. Referring to FIG. 1(e), a CANOST code linear notation rule adopting a predetermined linear notation rule as an example will be herein described.

In FIG. 1(e), symbol "*" denotes a boundary between adjacent levels, symbol ";" denotes a boundary between atoms belonging to the same hierarchy, and symbol "," denotes a boundary between atoms in one lower hierarchy to be connected. Furthermore, in FIG. 1(e), an under line drawn below C1 at node 2 denotes the position of an atom at which a cyclic structure is cut when the molecular tree is prepared.

Furthermore, the linear notation shown in FIG. 1(e) is a CANOST linear notation which is an example of a planar structure code expressing a planar structure of a molecule, and corresponds to "CANOST" in FIG. 20 and so forth which will be described later.

As can be clearly seen from the foregoing, the planar structure code according to the present invention is formed as follows. That is, as shown in FIG. 1(e), there is prepared a planar structure code expressing the planar structure of a molecule every start atom by carrying out the linear notation of the third molecular tree expressed using planar structure symbols, e.g., CANOST codes, which express the bonding relationship between a plurality of atoms as a planar structure, in accordance with a linear notation rule (a CANOST code linear notation rule) for expressing the third molecular tree by a row of characters.

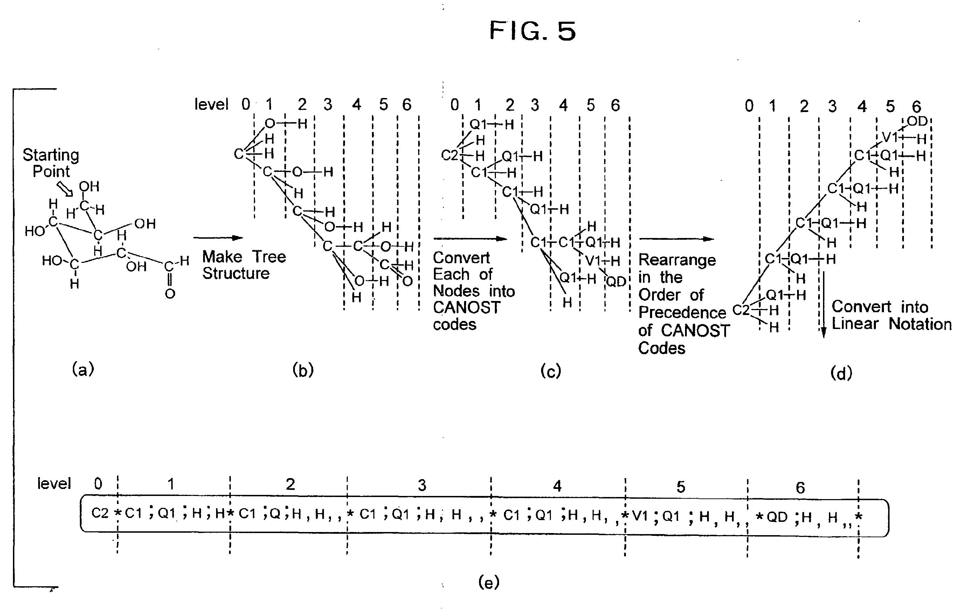

FIG. 5 shows a molecular tree and a CANOST linear notation which are prepared using a-Glc-4C-chain as a model molecule. In FIG. 5, a first molecular tree shown in (b) is formed from a conformation (a) of a-Glc-4C-chain. The first molecular tree is converted into CANOST codes to form a second molecular tree shown in (c). Then, a third molecular tree shown in (d) is formed from the second molecular tree using a CANOST code precedence rule. Moreover, a CANOST linear notation shown in (e) is obtained from the third molecular tree using a CANOST code linear notation rule. With respect to a-Glc-4C-chain, as compared with a-Glc-4C, its planar structure (planar structure) is shown in FIG. 12(b), and its configuration (configuration) is shown in FIG. 13(b).

A coding step according to the present invention will be described below.

FIG. 3 is an enlarged view of the molecular tree (third molecular tree) shown in FIG. 1(d). For example, in FIG. 3, "Q1" 11 in level 4 is noticed. Then, "C1" 12 in level 3, which is to be combined with the "Q1" 11 in level 3, is extracted. The atom in level 3, which is to be combined with the "Q1" 11, is uniquely determined to be the "C1" 12. Then, "C1" 13 to be combined with the "C1" 12 in level 2 is extracted. Similarly, the atom in level 2, which is to be combined with the "C1" 12, is uniquely determined to be the "C1" 13. Then, "C1" 14 to be combined with the "C1" 13 in level 1 is extracted. Similarly, the atom in level 1, which is to be combined with the "C1" 13, is uniquely determined to be the "C1" 14. Thus, there is set one group comprising four atoms which comprise the "Q1" 11 in level 4, the "C1" 12 in level 3, the "C1" 13 in level 2, and the "C1" 14 in level 1.

It should be herein noted that the group comprising the four atoms is uniquely determined if an atom to be noticed (the "Q1" 11 in level 4 in the above described example) is determined since atoms to be combined are extracted from the higher hierarchy side to the lower hierarchy side in the molecular tree. Therefore, according to the present invention, the stereochemistry of the noticed atom can be uniquely expressed by a dihedral angle which will be described later.

Then, with respect to four groups comprising the "Q1" 11 in level 4, the "C1" 12 in level 3, the "C1" 13 in level 2 and the "C1" 14 in level 1, a dihedral angle is derived. The dihedral angle means an angle between a plane, which is formed by the "Q1" 11 in level 4, the "C1" 12 in level 3 and the "C1" 13 in level 2, and a plane which is formed by the "C1" 12 in level 3, the "C1" 13 in level 2 and the "C1" 14 in level 1. The value of the dihedral angle is obtained from information on the molecular three-dimensional structure of a-Glc-4C.

The dihedral angle derived with respect to the noticed "Q1" 11 in level 4 is replaced with an angular symbol, which is defined in accordance with a predetermined angle dividing rule, in accordance with the magnitude of the dihedral angle, and the replaced angle symbol is given to the noticed "Q1" 11 in level 4.

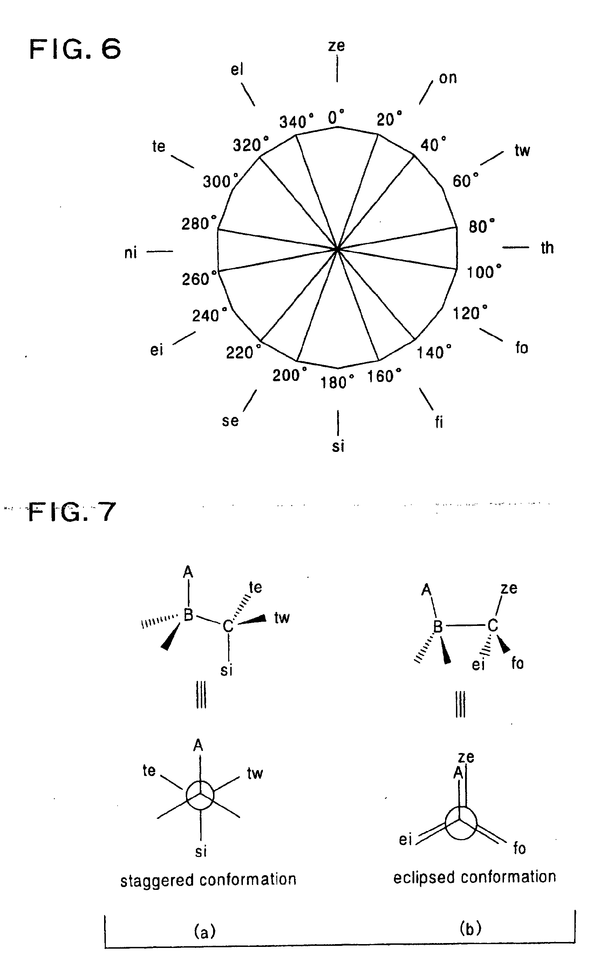

Referring to FIG. 6, an example of a predetermined angle dividing rule will be described below. In the angle dividing rule adopted herein, an angle of 360 degrees is divided into 12 angular ranges in the form of a clock dial. To the center of each of the divided angular ranges, a symbol corresponding to a figure of the clock dial is added. As shown in FIG. 6, 12 angular symbols including ze (zero), on (one), tw (two), th (three), fo (four), fi (five), si (six), se (seven), ei (eight), ni (nine), te (ten) and el (eleven) are provided in the angular ranges of one o'clock, two o'clock, three o'clock, four o'clock, five o'clock, six o'clock, seven o'clock, eight o'clock, nine o'clock, ten o'clock and eleven o'clock, respectively. By these 12 angular symbols, corresponding angular ranges are expressed.

The angle division shown in FIG. 6 is mainly based on the abundance ratio in a steric structure taken by atoms of sp³ type. That is, to a staggered conformation (such as a stable conformation of a chain molecule or a six-membered ring) shown in FIG. 7(a), wide angular ranges of 40 degrees about the angular centers of 60, 180 and 300 degrees are given, and angular symbols of tw, si and te are added, respectively. In addition, to an eclipsed conformation (such as a three-membered ring or a bicyclo ring) shown in FIG. 7(b) which has the second greatest abundance ratio, wide angular ranges of 40 degrees about the angular centers of 0, 120 and 240 degrees are given, and angular symbols of ze, fo and ei are added, respectively. To other angular ranges about the angular centers of 90, 210, 330, 30, 150 and 270 degrees, six angular symbols of th, se, el, on, fi and ni are given, respectively.

Furthermore, with respect to other atoms which are included in alkyne, allene and or the like and which can not define any dihedral angles, special codes are set therefor to give symbols thereto, so that the linear notation of the molecular tree can be carried out in the same manner as that in the above described case where the angular symbols are given. With respect to atoms of sp² type and sp type, codes therefor can be set and expressed.

According to the angle dividing rule shown in FIG. 6, on the basis of information on the molecular three-dimensional structure of a-Glc-4C, an angular symbol "si" is given to the above described noticed "Q1" 11 in level 4.

Similarly, as described above, a dihedral angle is derived with respect to a group comprising four atoms extending over four levels, and an angular symbol is given thereto.

Furthermore, atoms belonging to level 2, level 1 or level 0 can not form a group comprising four atoms even in a lower hierarchy. Therefore, symbols of "A", "B" and "C" are uniformly given to atoms in level 0, level 1 and level 2, respectively, as shown in "Stereo" in FIG. 20 and so forth.

Thus, using angular symbols of "A", "B", "C" and "ze", ···, "el", the molecular tree shown in FIG. 3 is replaced with the angular symbols.

A conformation code preparing step according to the present invention will be described below.

The linear notation of the molecular tree coded by the angular symbols is carried out in accordance with the CANOST code linear notation rule so as to correspond to the molecular tree. Then, conformation codes using the atom at the node 6 of a-Glc-4C as a start atom are prepared. The prepared conformation codes using the atom at the node 6 of a-Glc-4C as the start atom are shown at the position of number "6" in the left column of "Stereo" in FIG. 20.

Similarly, another atom of a-Glc-4C is used as a start atom for preparing conformation codes which are shown at the position of number "1" and so forth in the left column of "Stereo" in FIG. 20.

As described above, the conformation codes are derived by the hierarchy classifying step, molecular tree forming step, coding step and conformation code preparing step. In FIGS. 20 through 29, conformation codes (expressed as "Stereo") derived with respect to various molecules are shown. Furthermore, in FIGS. 20 through 29, CANOST linear notations which are planer structure codes are described as "CANOST", and configuration codes which will be described later are described as "Configuration".

Furthermore, at the conformation code preparing step, even if atoms at all of the nodes are used as start atoms, if conformation codes are prepared using two start atoms, the difference between the numbers of the hierarchies of which is three or more, the stereochemistry of the whole molecule can be expressed. Thus, even if atoms at all of the nodes are not adopted as start atoms, if only atoms noticed with respect to stereochemistry or atoms near thereto are adopted as start atoms to derive conformation codes corresponding thereto, it is possible to sufficiently know required stereochemistry.

The reason why the difference between the numbers of the hierarchies of two start atoms is three or more is that it is possible to obtain all of dihedral angles including atoms in level 0, level 1 and level 2.

At the molecular tree forming step, a group of atoms having a low degree of notice in the identification of stereochemistry can be replaced with predetermined symbols to be masked. Thus, the stereochemistry of a group of atoms having a high degree of notice in the identification of stereochemistry can be directly compared.

Another preferred embodiment of the present invention will be described below.

According to the present invention, configuration codes expressing the configuration of a molecule can be prepared. A configuration code preparing step of preparing configuration codes will be described below.

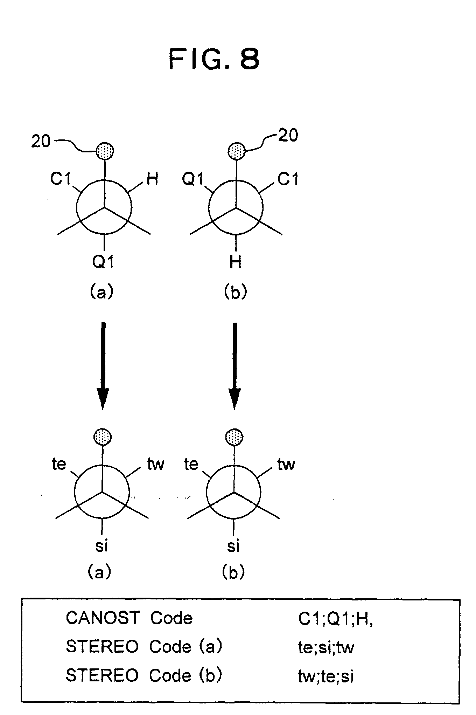

FIG. 8 is a diagram showing a conformation code preparing step by a Newman projection drawing. This figure shows three atoms C1, H and Q1 which are combined with one atom and which are shown by codes C1, H and Q1 belonging to the (n+3)-th hierarchy, and an atom 20 belonging to the n-th hierarchy which is lower than the hierarchy of the three atoms by three hierarchies. The atom 20 is positioned at a reference position, e.g., "ze" of 0 o'clock. Then, an angle required to rotate each of the three atoms C1, H and Q1 belonging to the (n+3)-th hierarchy counterclockwise around a bonding axis, which connects an atom in the (n+1)-th hierarchy to an atom in the (n+2)-th hierarchy, to cause each of the three atoms to overlap with the atom 20 is the magnitude of a dihedral angle. That is, the angle between a plane, which is formed by each of the three atoms C1, H and Q1 belonging to the (n+3)-th hierarchy and the bonding axis connecting the atom in the (n+1)-th hierarchy to the atom in the (n+2)-th hierarchy, and a plane, which is formed by the atom 20 in the n-th hierarchy and the bonding axis connecting the atoms in the (n+1)-th hierarchy and the atoms in the (n+2)-th hierarchy, viewed clockwise from the atom 20 is the magnitude of a dihedral angle. As can be clearly seen from the stereostructure shown in FIG. 8, in the left projection drawing (a) and the right projection drawing (b), although it is common in both molecules that the three atoms belonging to the (n+3)-th hierarchy are C1, H and Q1, the conformation about the bonding axis connecting the atom in the (n+1)-th hierarchy and the atom in the (n+2)-th hierarchy is different. This is shown by the fact that the conformation codes of the molecule (a) are different from those of the molecule (b), referring to the conformation codes (herein expressed as STEREO codes) in FIG. 8.

However, it is not possible to know the difference between the configurations in the projection drawings (a) and (b) using only information shown in FIG. 8. Therefore, a configuration code preparing step capable of identifying the difference between the configurations of molecules will be described below.

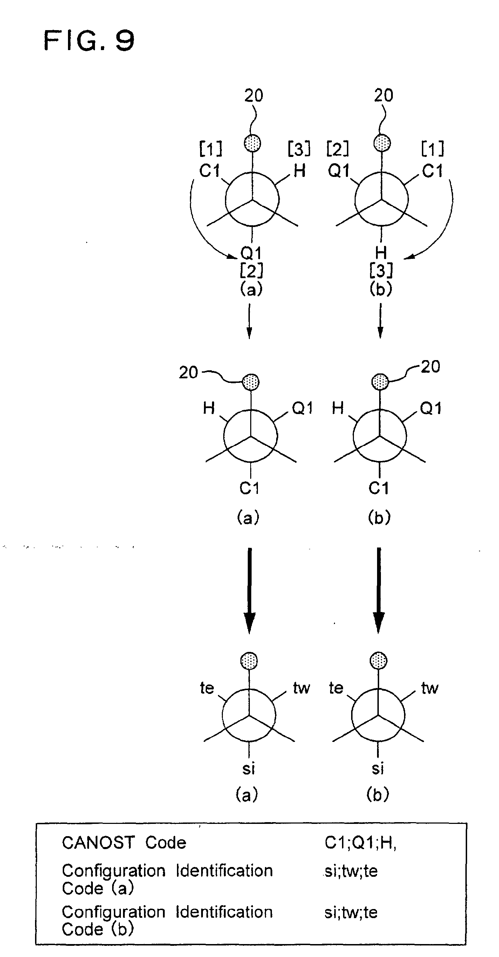

In FIG. 9, a most preferential atom (C1) between the three atoms C1, H and Q1 belonging to the (n+3)-th hierarchy is noticed in accordance with the above described CANOST code precedence rule. Then, all of the three atoms C1, H and Q1 belonging to the (n+3)-th hierarchy are integrally rotated around the bonding axis connecting the atom in the (n+1)-th hierarchy and the atom in the (n+2)-th hierarchy so that the most preferential C1 is arranged at an angular position of "si" which is an angular position of 180 degrees with respect to a reference position "ze". After this rotation, an angular symbol is given to each of the atoms C1, H and Q1 belonging to the (n+3)-th hierarchy in accordance with the angular position with respect to the reference position "ze". It can be seen from FIG. 9 that configuration codes (herein expressed as configuration identification codes) in the projection drawing (a) are the same as those in the projection drawing (b), so that the configurations are the same.

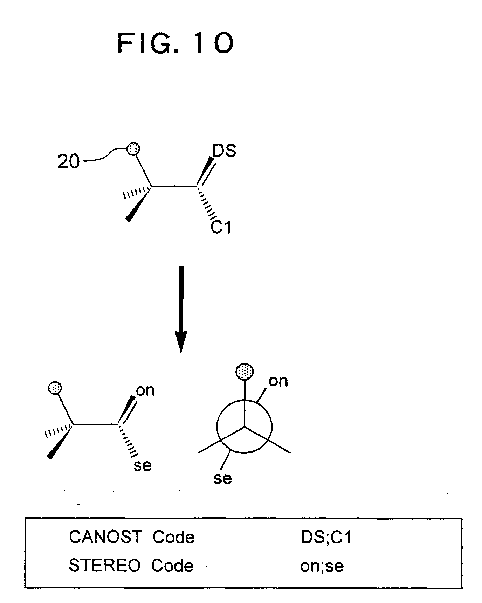

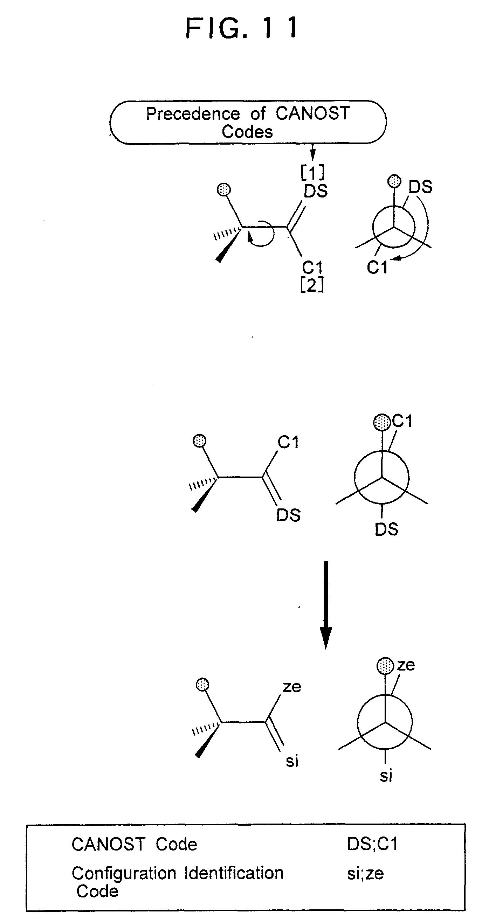

FIGS. 10 and 11 are diagrams for explaining a conformation and configuration with respect to a single bond of sp² type.

FIG. 10 shows atoms which belong to the (n+3)-th hierarchy and which are denoted by codes of DS and C1, and an atom 20 belonging to the n-th hierarchy which is lower than the hierarchy of the atoms by three hierarchies. The atom 20 is positioned at the reference position "ze". Then, each of the two atoms DS and C1 belonging to the (n+3)-th hierarchy is rotated counterclockwise about the bonding axis connecting atoms (not shown) in the (n+1)-th hierarchy to atoms in the (n+2)-th hierarchy, to derive a dihedral angle which serves as an angle required to cause each of the atoms to overlap with the atom 20. FIG. 10 shows conformation codes (herein expressed as STEREO codes) which are derived in the same manner as that in FIG. 8. Since the atoms DS and C1 belonging to the (n+3)-th hierarchy are arranged on the same plane as those of the atoms belonging to the (n+1)-th hierarchy and the atoms belonging to the (n+2)-th hierarchy, the code of DS is "on", whereas the code of C1 is "se" which is arranged at an angular position shifted from "on" by 180 degrees.

In FIG. 11, with respect to a single bond of atoms of sp² type, configuration codes (herein expressed as configuration identification codes) are derived in the same manner as that in FIG. 9.

With respect to a double bond portion of atoms of sp² type, the atoms belonging to the (n+3)-th hierarchy can not be rotated about the bonding axis connecting the atoms in the (n+1)-th hierarchy to the atoms in the (n+2)-th hierarchy. Therefore, angular symbols in conformation codes may be adopted as they are, so that configuration codes may be prepared.

With respect to atoms of sp type, the conformation and configuration thereof can be expressed using special codes which express special structures such as a triple bond, a portion surrounding the triple bond or allene.

As described above, the configuration codes expressing the configuration of a molecule can be prepared by the configuration code preparing step. The configuration codes (herein expressed as Configuration) derived with respect to various molecules are shown in FIGS. 20, 25, 28 and 29.

An example of a molecule to which the present invention is applied will be described below.

FIG. 20 shows CANOST linear notations (herein expressed as CANOST) which are planar structure codes, conformation codes (herein expressed as Stereo) and configuration codes (herein expressed as Configuration) with respect to a-Glc-4C.

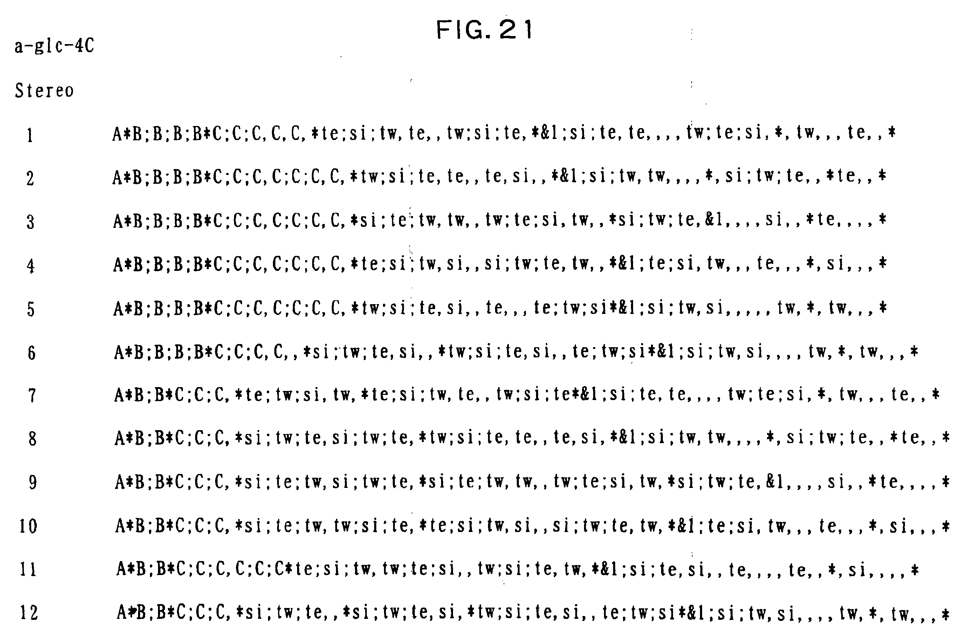

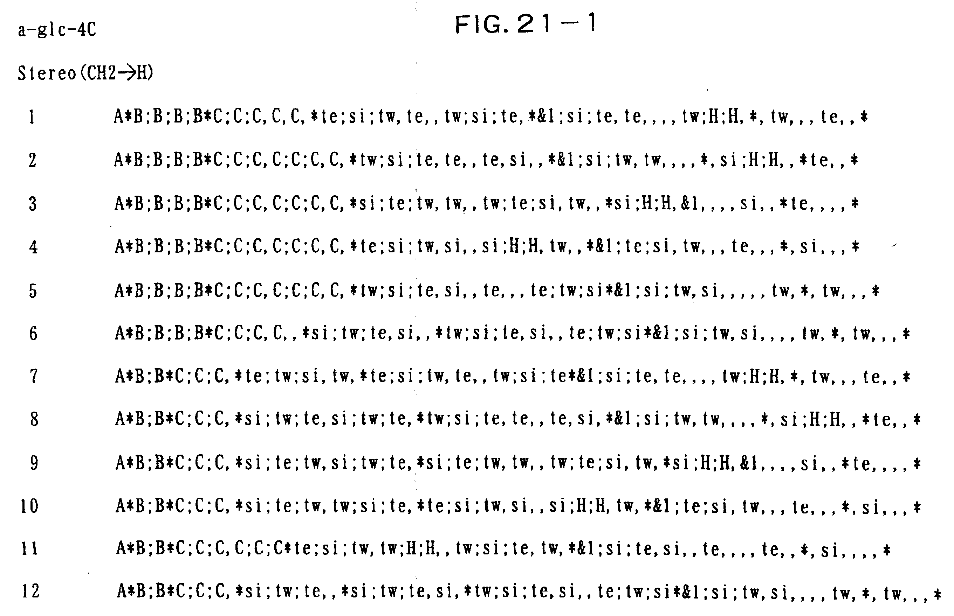

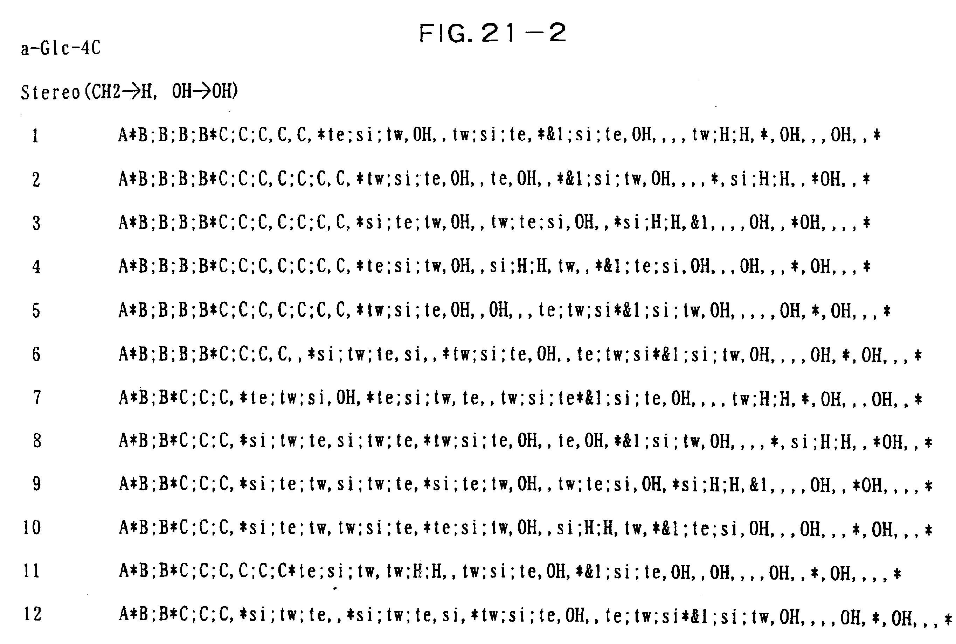

FIG. 21 shows conformation codes (Stereo) derived by masking a group of atoms having a low degree of notice in the identification of stereochemistry. In this figure, the top stage includes conformation codes which are not masked, the middle stage includes conformation codes which are obtained by replacing each of hydrogen atoms of a methylene group CH₂ with a code "H" and masking the replaced codes, and the bottom stage includes conformation codes which are obtained by replacing each of hydrogen atoms of a methylene group CH₂ with a code "H", replacing the hydrogen atom of a hydroxyl group OH with a code "OH" and masking the replaced codes.

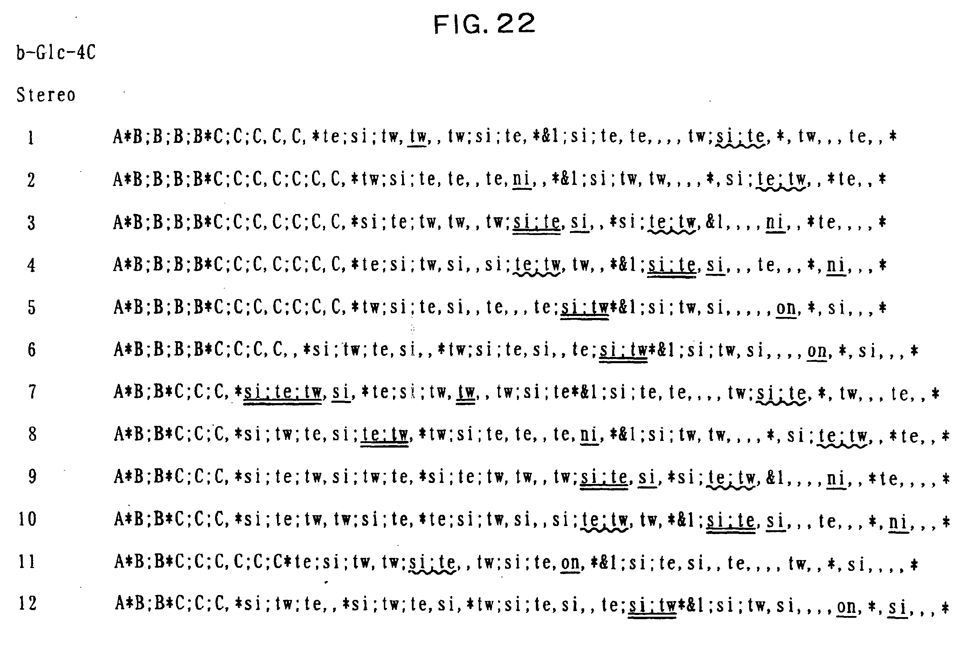

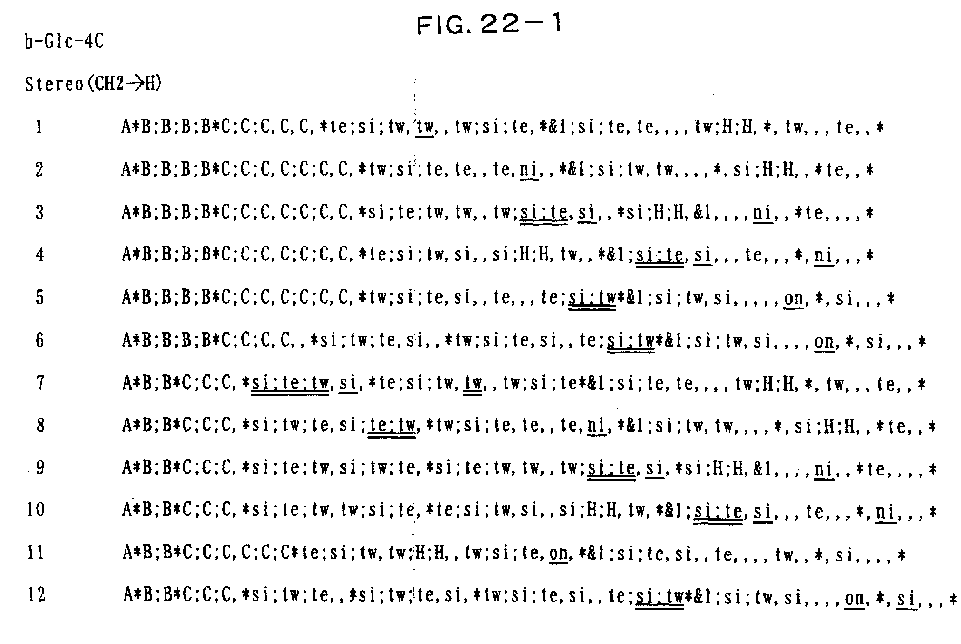

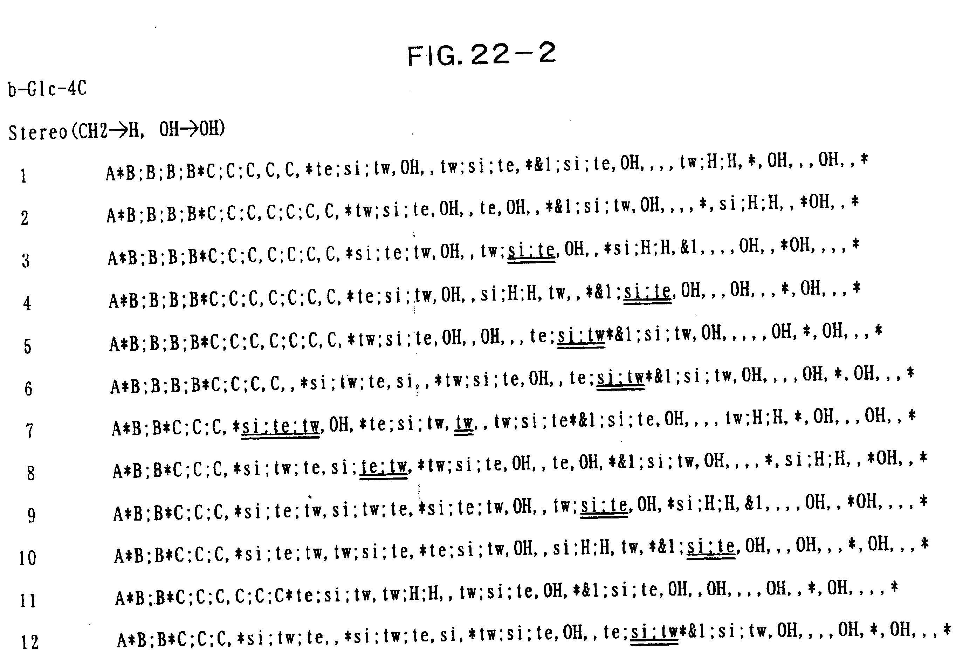

FIG. 22 shows conformation codes (herein expressed as Stereo) of b-Glc-4C which is an object to be compared with a-Glc-4C. In this figure, the top stage includes conformation codes which are not masked, the middle stage includes conformation codes which are obtained by masking each of hydrogen atoms included in a methylene group CH₂, and the bottom stage includes conformation codes obtained by masking atoms, which are included in a methylene group CH₂ and hydroxyl group OH, by the above described manner.

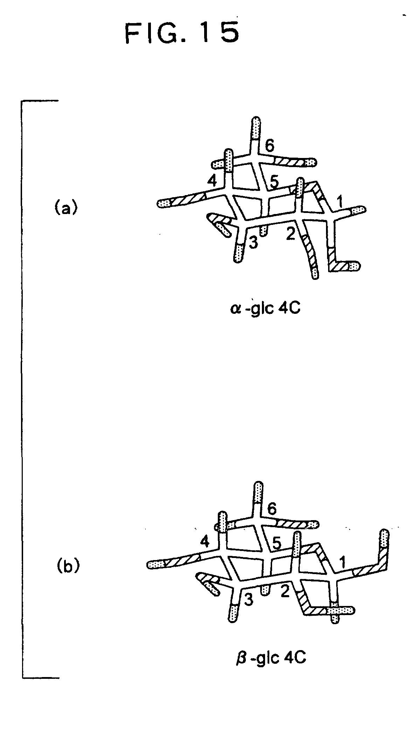

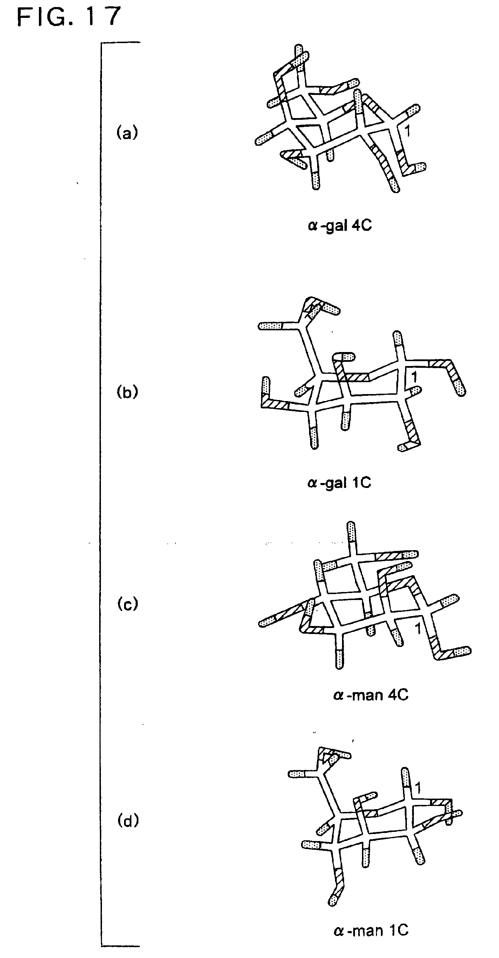

FIG. 15 shows stereostructural formulae of a-Glc-4C and b-Glc-4C. As described above, with respect to stereochemistry at the 1-position, one having "a-", such as a-Glc, shows a configuration of α-Glc, and one having "b-" shows a configuration of β-Glc. Furthermore, in FIGS. 15 through 18, portions shown by slant lines denotes oxygen, portions shown by a void denotes carbon, and portions shown by sands denotes hydrogen atoms.

Referring to FIGS. 20, 21 and 22, the conformation codes (Stereo) of a-Glc-4C and b-Glc-4C will be compared with each other.

Although the two molecules of a-Glc-4C and b-Glc-4C have a common conformation 4C of a carbon skeleton, the stereochemistry at the 1-position thereof is different from each other. In addition, the direction of hydrogen of a hydroxyl group at the 2-position is greatly different.

In FIG. 22, portions shown by double under lines are portions to which different codes are assigned in accordance with the difference in stereochemistry at the 1-position. By noticing the portions shown by the double under lines, it is possible to know that the stereochemistry at the 1-position of a-Glc-4C is different from that of b-Glc-4C.

In addition, portions shown by single under lines are portions showing the difference between the orientations of hydroxyl groups. In order to know the difference between the orientations of hydroxyl groups, the portions shown by the single under lines may be noticed.

Moreover, portions shown by wave under lines are produced by the inversion of the precedence of the CANOST code precedence rule, which is caused since serial numbers of two hydrogen atoms of a methylene (CH₂) at the 6-position are different when a structure is inputted. In such a case, as described above, they are replaced with other angular symbols to be masked. Thus, it is possible to ignore the difference, which is not based on the essential structural difference, to effectively examine the presence of the considerable difference of the structure.

Furthermore, although there are some cases where it is required to identify the orientation of a hydroxyl group, only the conformations of basic skeletons ignoring this are generally often compared. In such cases, a code corresponding to hydrogen of the hydroxyl group is masked with "OH".

As a start atom, hydrogen is omitted, node numbers 1 through 6 correspond to C-1 through C-6, and node numbers 7 through 12 correspond to O-1 through O-6. Although each of a-Glc-4C and b-Glc-4C is an epimer at the 1-position, all of codes other than those starting from node numbers 1, 2 and 11 are partially different. Since dihedral angle information about a asymmetric center at the 1-position does not exist in conformation codes (Stereo) starting from the vicinity of a asymmetric center at the 1-position, it is possible to identify a molecule by the conformation codes (Stereo) of a start atom which is spaced from the asymmetric center. Furthermore, as described above, the conformation of the whole molecule can be expressed by the conformation codes of two start atoms which are generally spaced by three bonds, and stereoisomerism can be identified.

According to FIG. 22, different portions of conformation codes (Stereo) of b-Glc-4C from those of a-Glc-4C can be quite obviously known with respect to cases where no masking is carried out (top stage) and where a masking is carried out (middle and bottom stages).

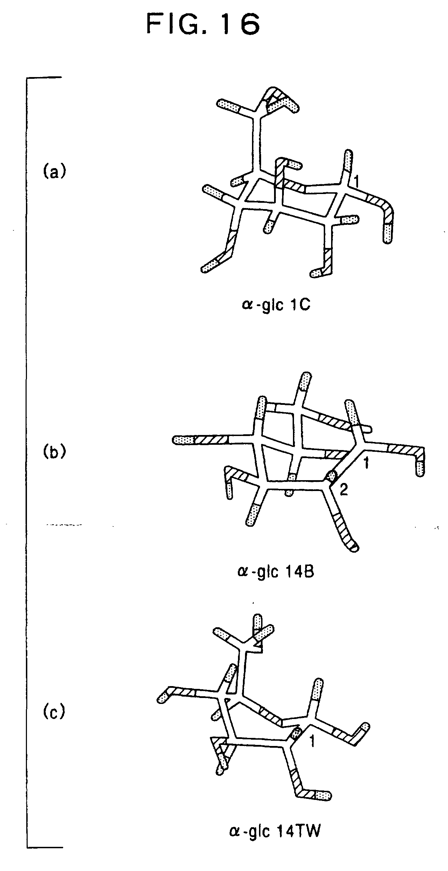

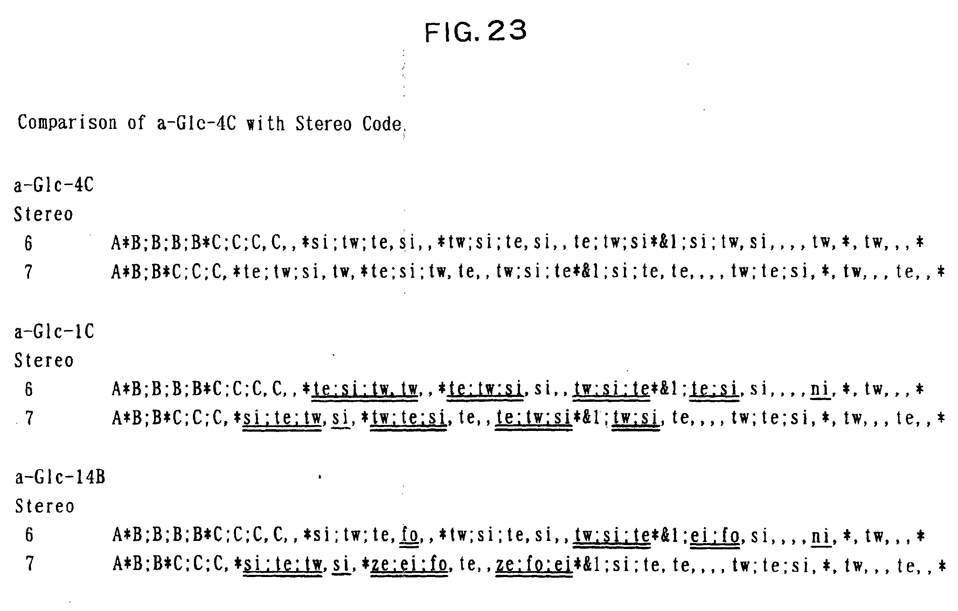

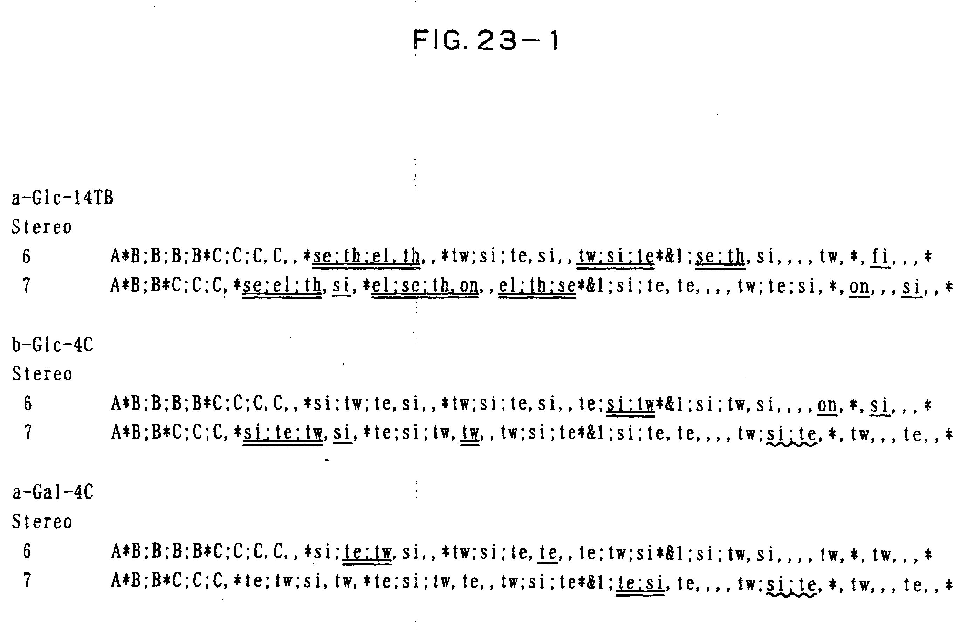

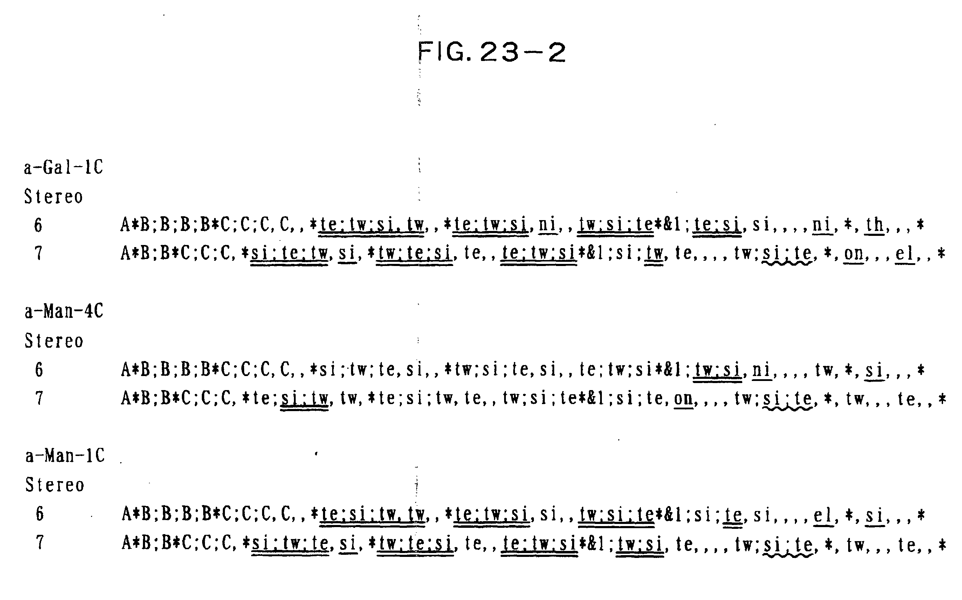

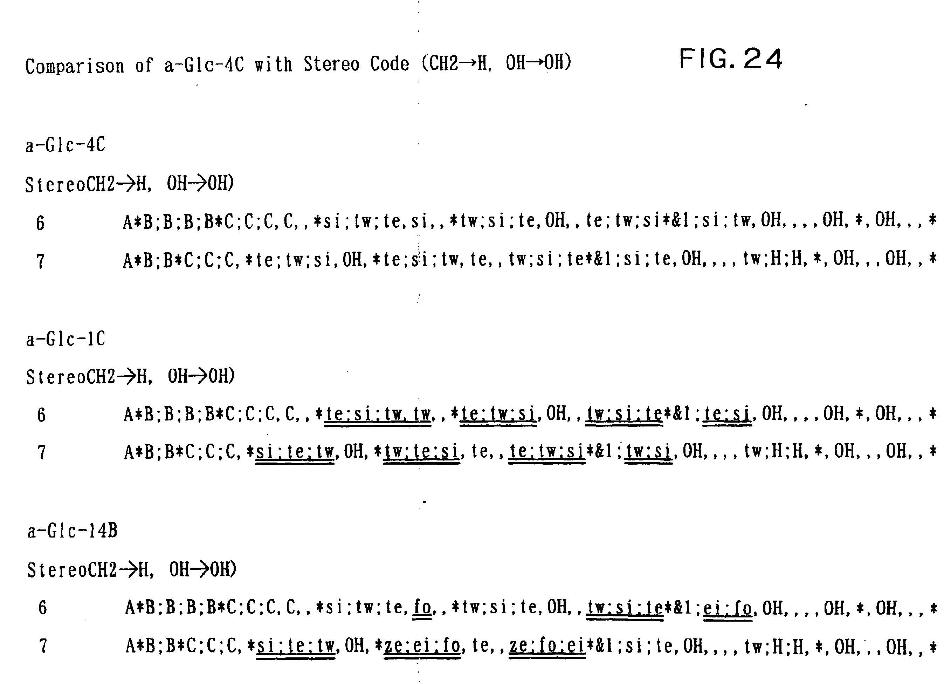

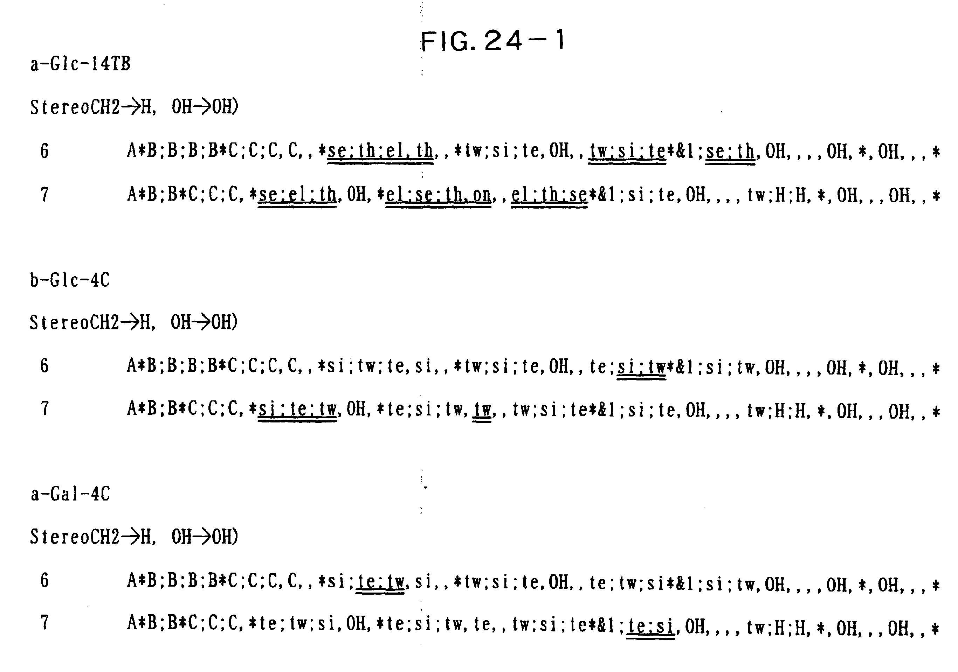

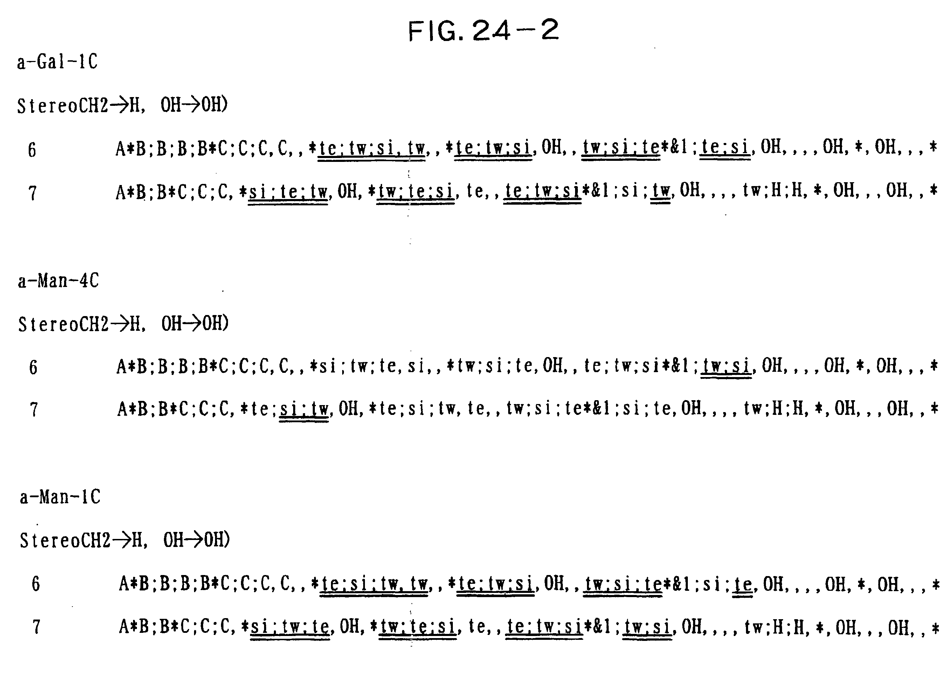

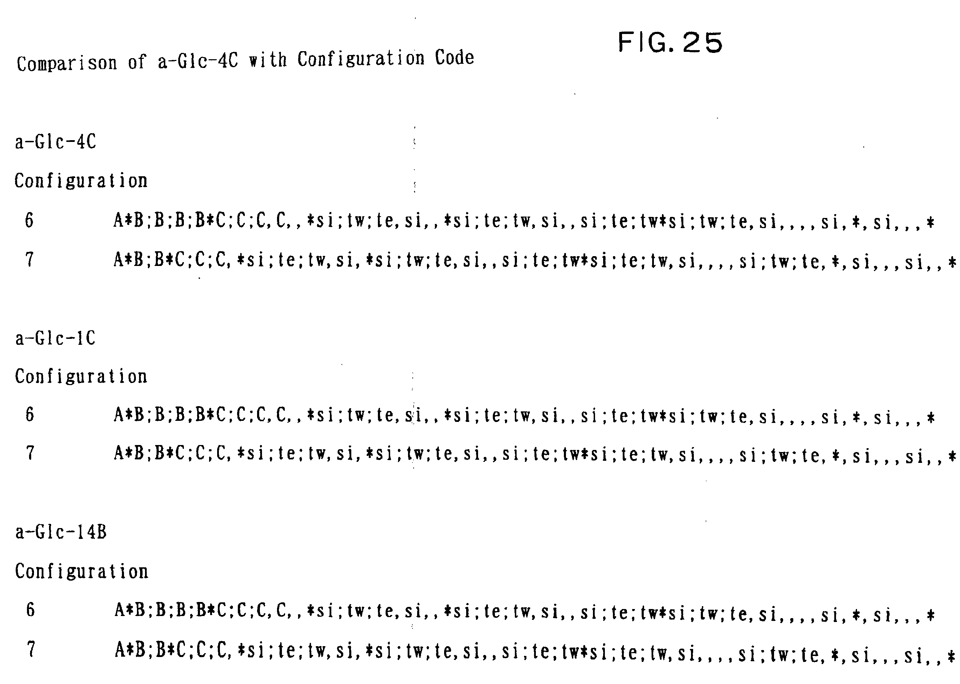

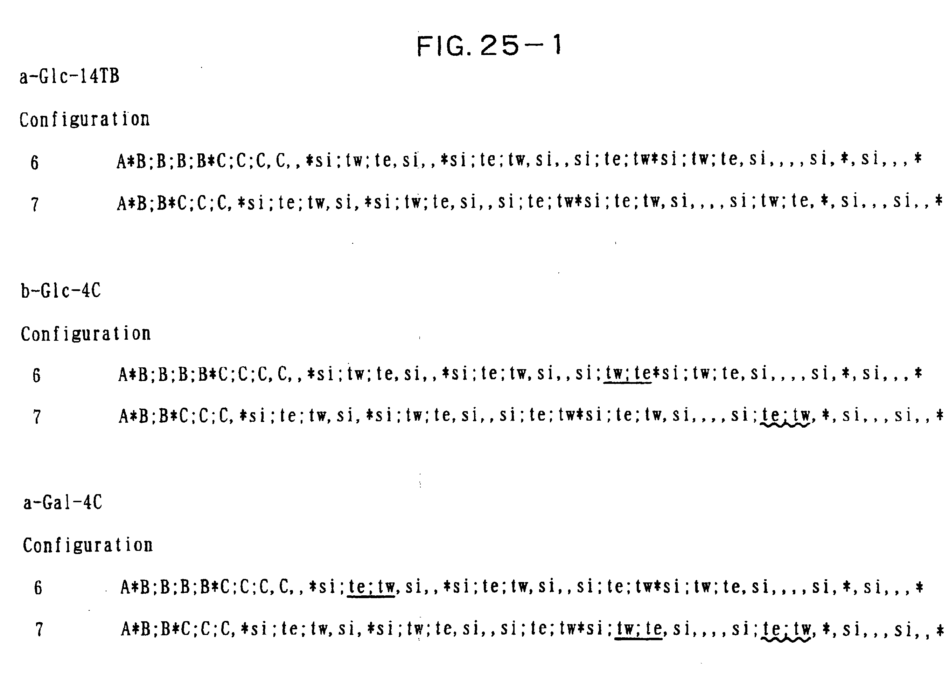

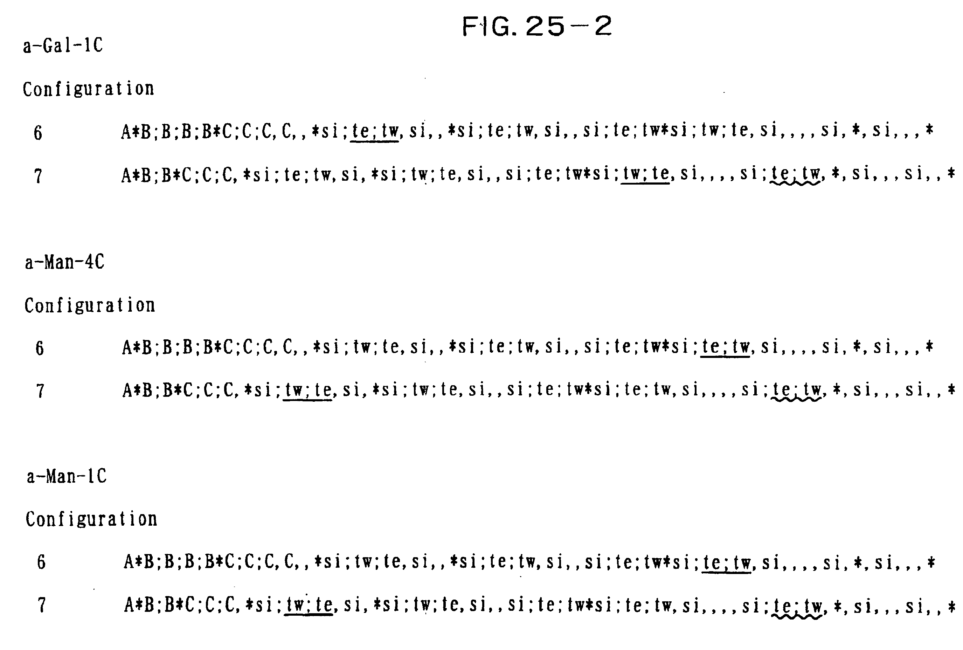

FIGS. 23 through 25 show the comparison of the obtained results at only two nodes 6 and 7 which are selected as start atoms with respect to various molecules, the stereostructural formulae of which are shown in FIGS. 15 and 16. In FIG. 23, conformation codes (Stereo) which are not masked are compared. In FIG. 24, conformation codes (Stereo) which are masked are compared. In FIG. 25, configuration codes (Configuration) are compared. In all of the figures, the comparison is carried out with respect to a-Glc-4C, and different portions are shown by single and double under lines similar to the above described case.

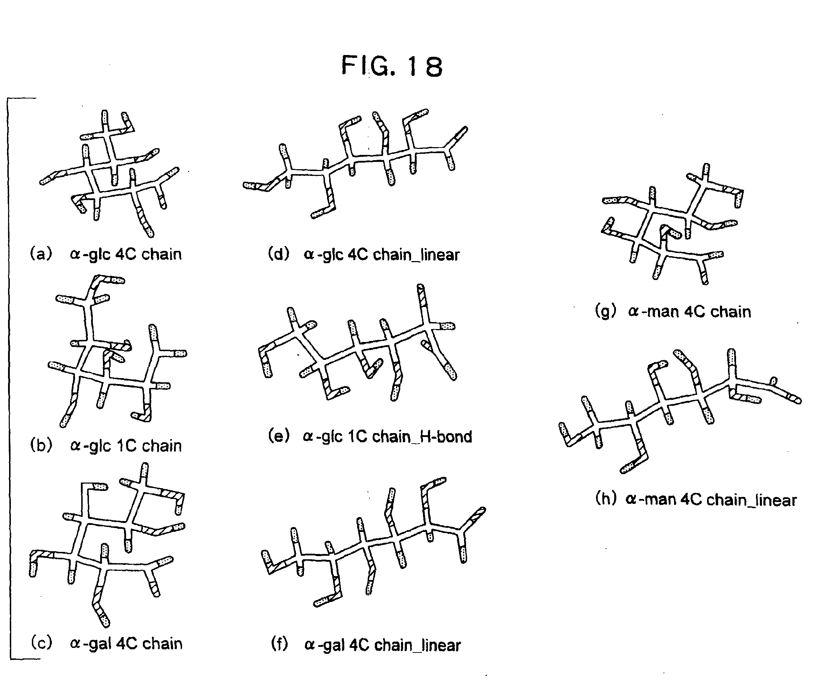

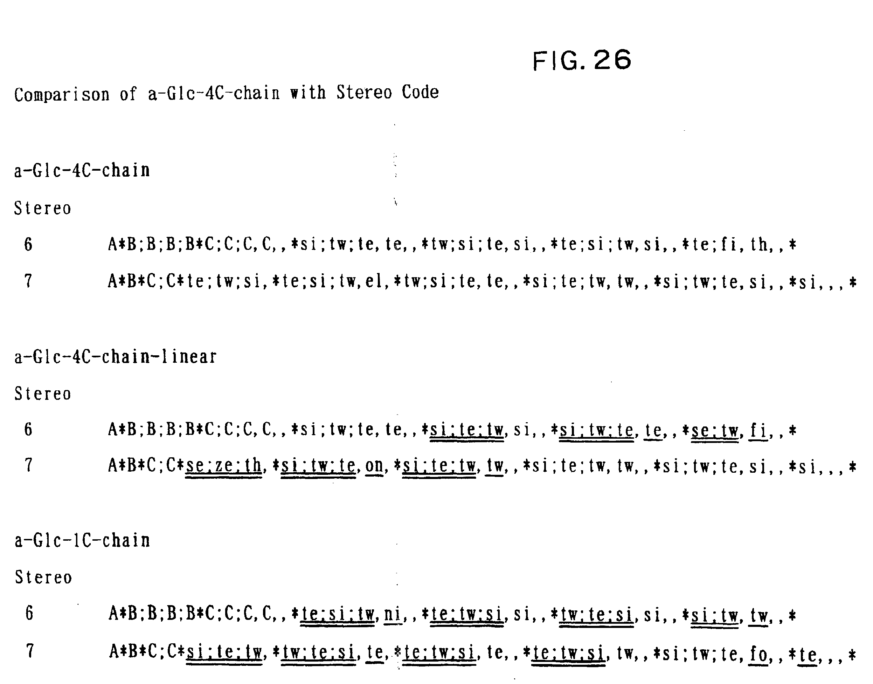

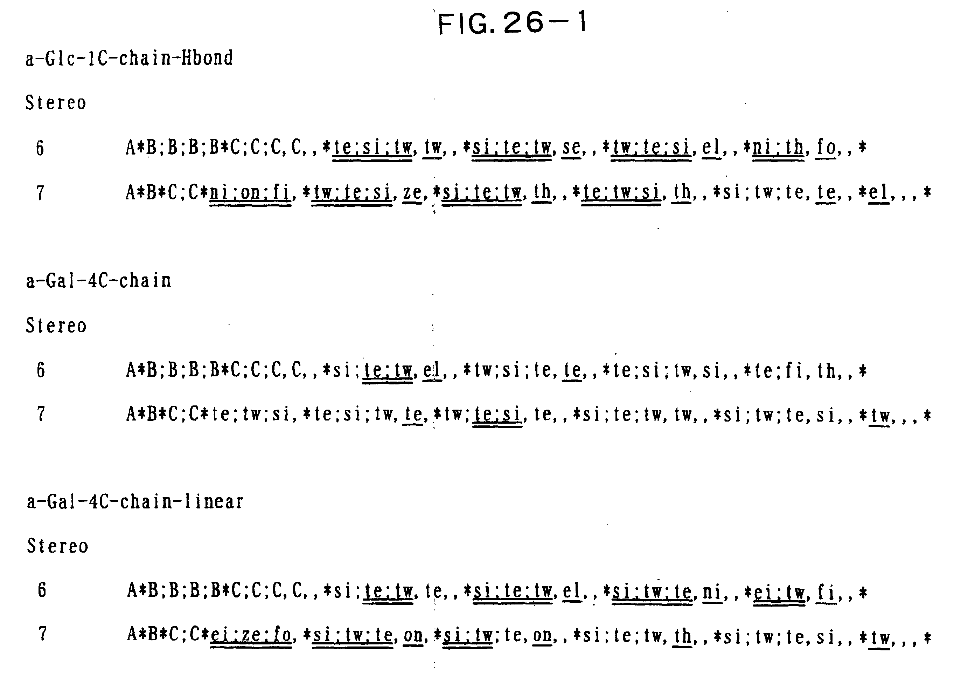

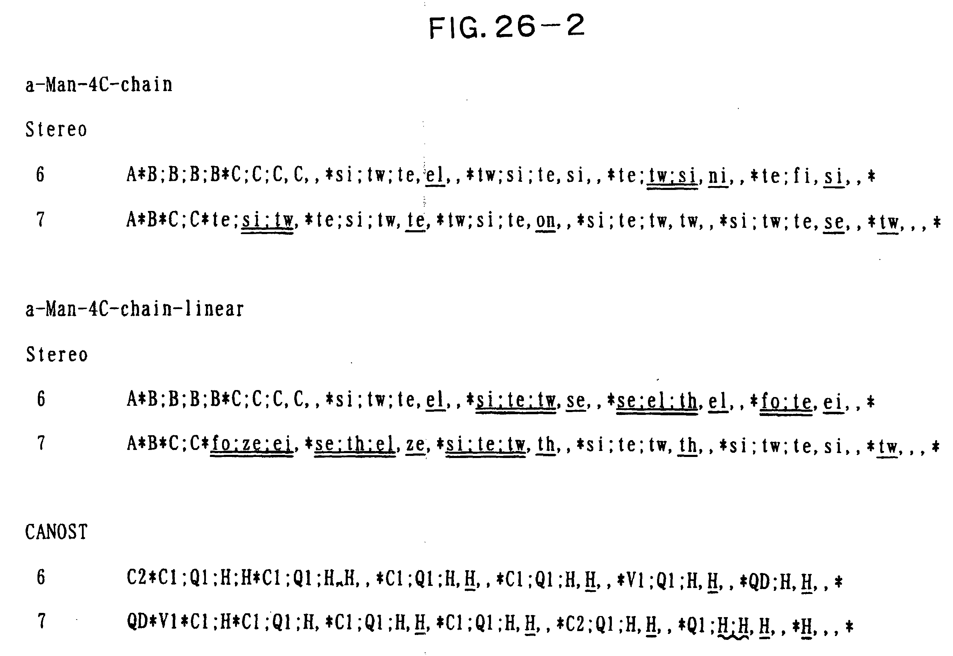

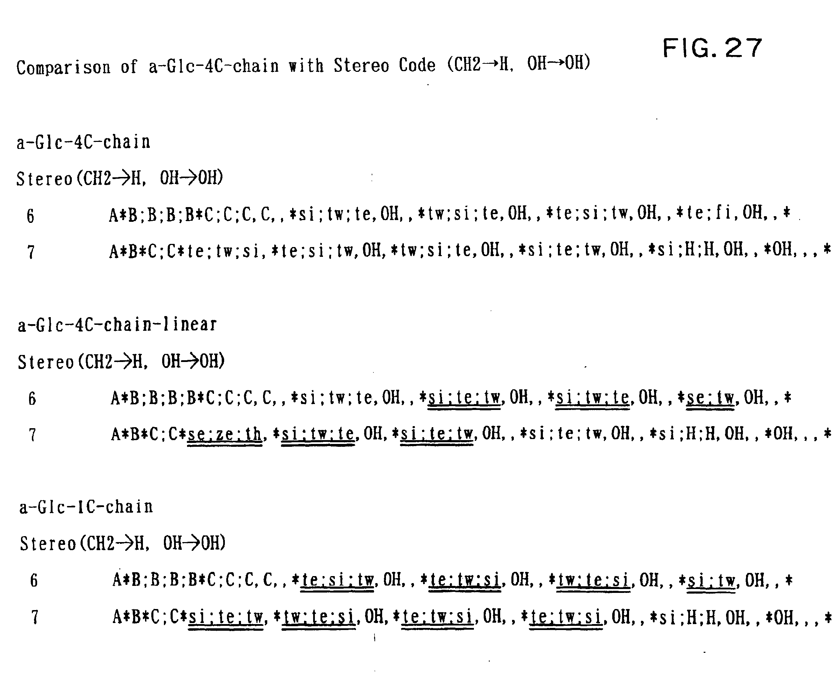

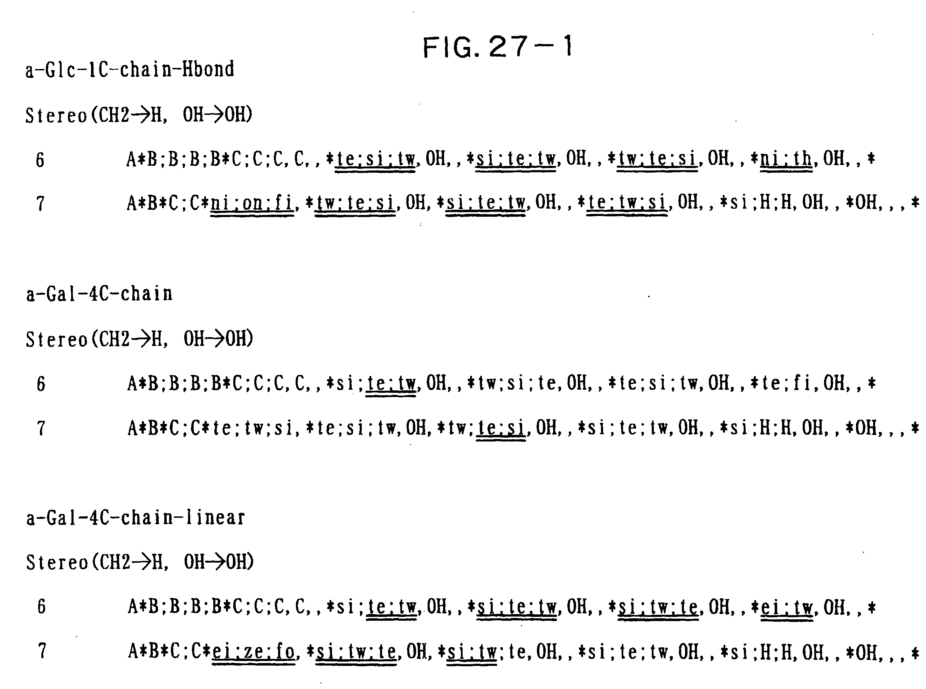

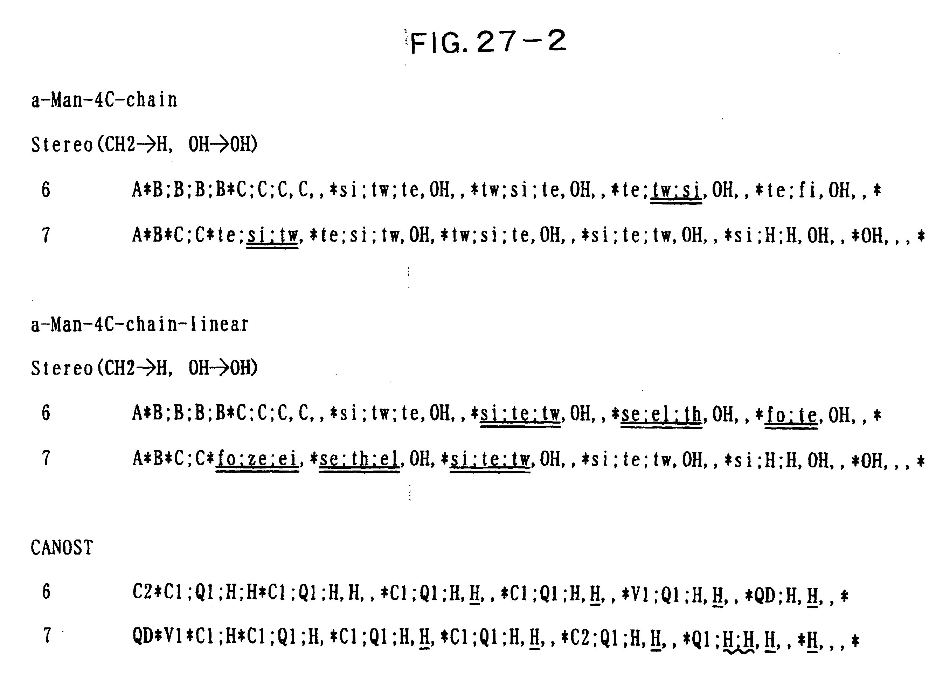

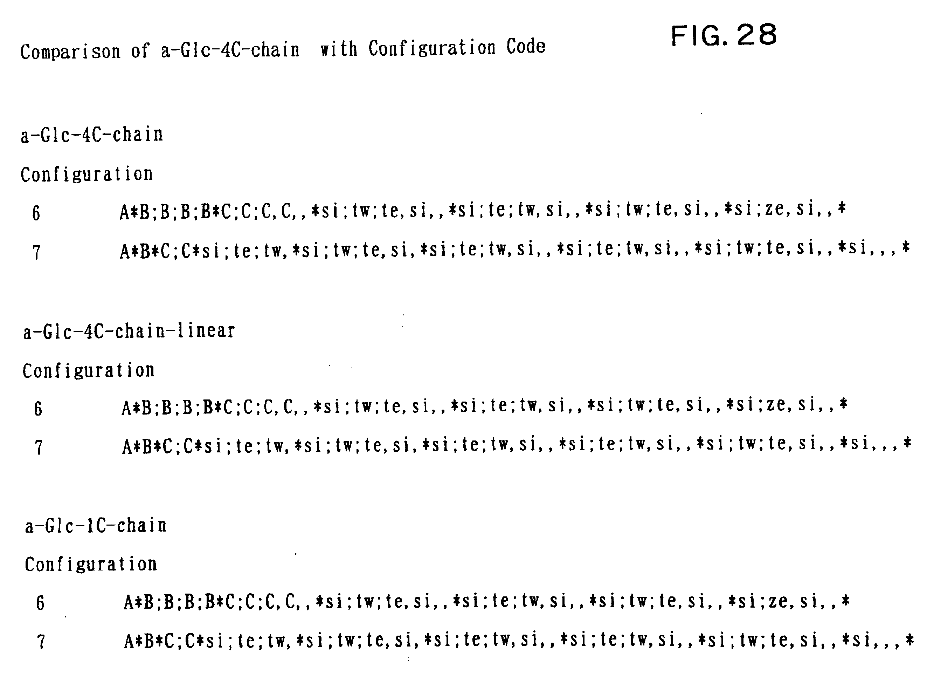

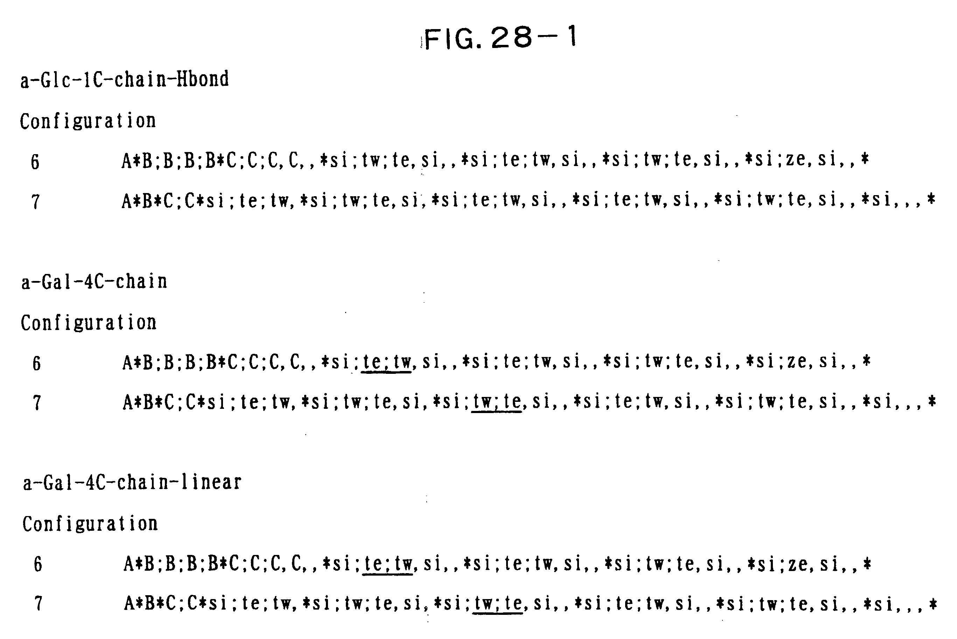

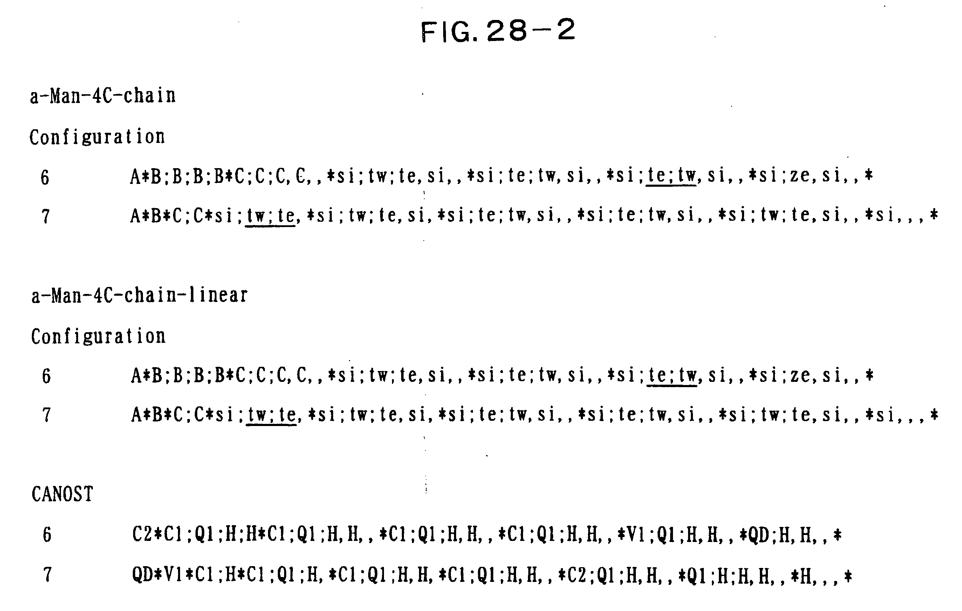

FIGS. 26 through 28 show the comparison of the obtained results at only two nodes 6 and 7 which are selected as start atoms with respect to various molecules, the stereostructural formulae of which are shown in FIG. 18. FIG. 12(b) shows the planar structural formula of a molecule. In FIG. 26, conformation codes (Stereo) which are not masked are compared. In FIG. 27, conformation codes (Stereo) which are masked are compared. In FIG. 28, configuration codes (Configuration) are compared. In all of the figures, the comparison is carried out with respect to a-Glc-4C-chain, and different portions are shown by single and double under lines.

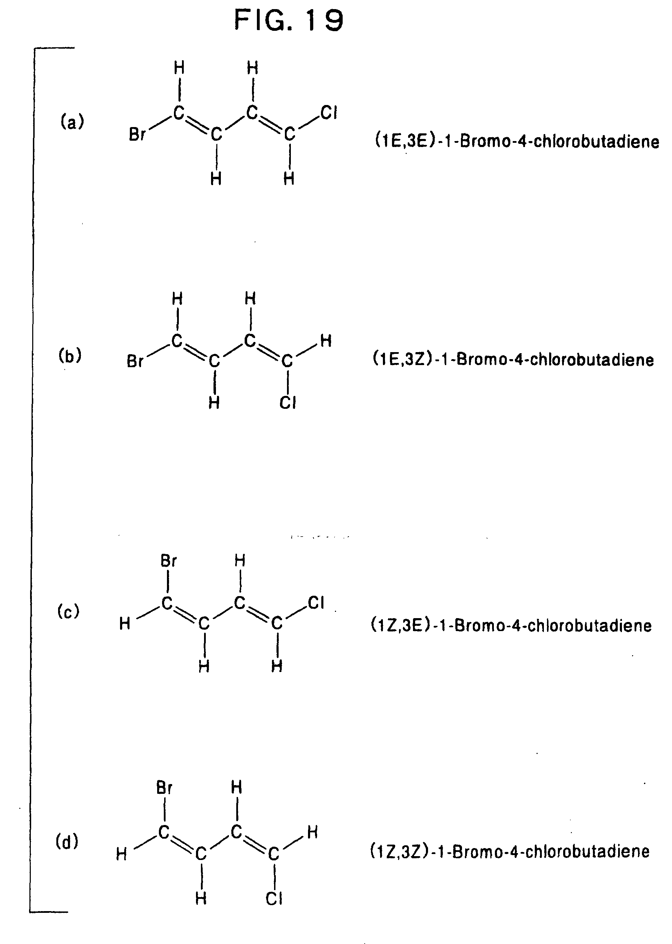

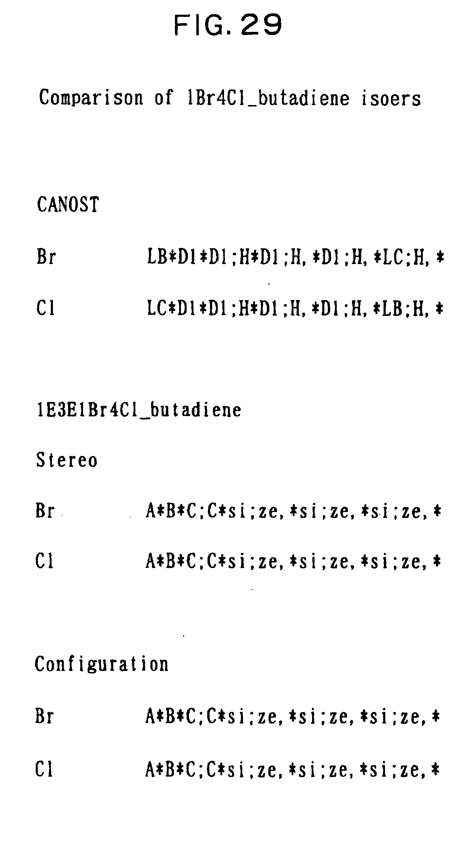

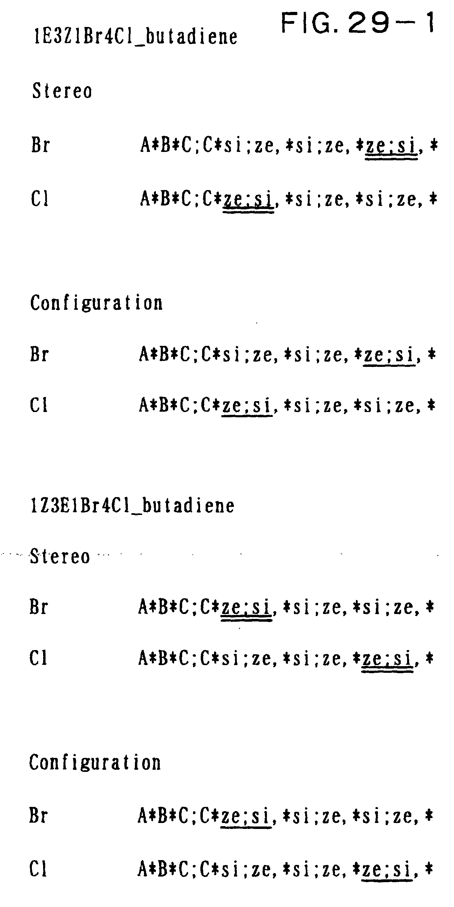

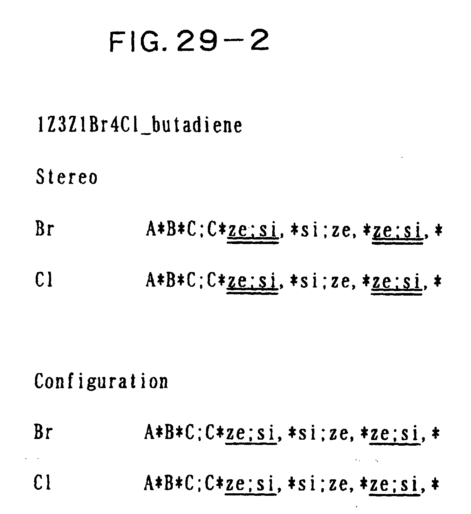

In FIG. 29, only two of Br and Cl atoms are selected as start atoms with respect to various molecules, the chemical formulae of which are shown in FIG. 19, and conformation codes (Stereo) and configuration codes (Configuration) are compared. In this figure, the comparison is carried out with respect to (1E, 3E)-1-bromo-4-chlorobutadiene, and different portions are shown by single and double under lines.

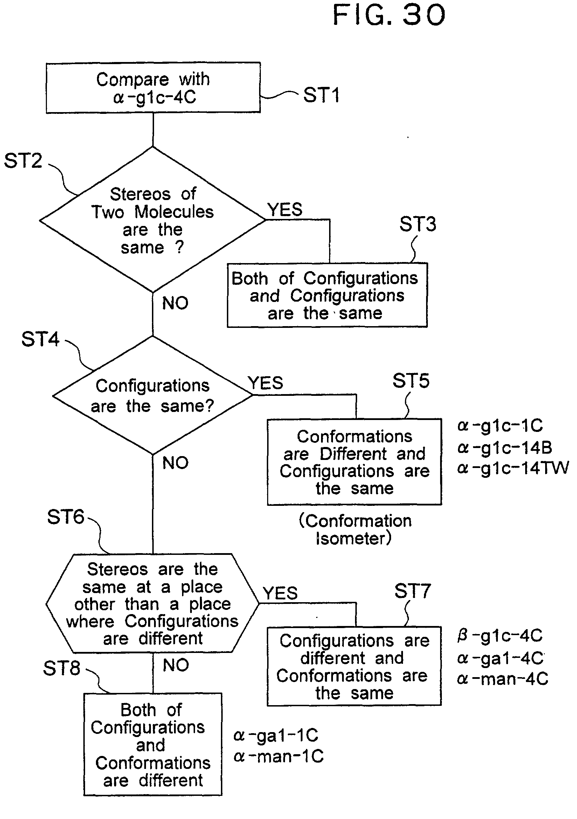

Referring to FIG. 30, an example of the use of conformation codes (Stereo) and configuration codes (Configuration) will be described below.

As described above, with respect to various molecules, the stereostructural formulae of which are shown in FIGS. 15, 16 and 17, conformation codes (Stereo) and configuration codes (Configuration) were obtained in FIGS. 23 through 25.

At ST1 of FIG. 30, data shown in FIGS. 23 through 25 are used for comparing stereochemistry between a-Glc-4C and molecules shown in FIGS. 15 and 16.

If it is determined at ST2 that the conformation codes (Stereo) are the same, it is determined at ST3 that both have the same conformation and configuration.

If it is determined at ST2 that the conformation codes (Stereo) are different, it is determined at ST4 whether the configuration codes (Configuration) are the same or different.

If it is determined at ST4 that the configuration codes (Configuration) are the same, it is determined at ST5 that both have different conformations and the same configuration, so that both are conformational isomers. With respect to a-Glc-4C, a-Glc-1C, a-Glc-14B and a-Glc-14TW correspond thereto.

If it is determined at ST4 that the configuration codes (Configuration) are different, it is determined at ST6 whether the conformation codes (Stereo) are the same in a place other than a place where the configuration codes (Configuration) are different.

If it is determined at ST6 that the conformation codes (Stereo) are the same in the place other than the place where the configuration codes (Configuration) are different, it is determined at ST7 that both have different configurations and the same conformation. More correctly, it is determined that the conformations at the place where the configuration codes (Configuration) are the same are the same. With respect to a-Glc-4C, b-Glc-4C, a-Gal-4C and a-Man-4C correspond thereto.

If it is determined at ST6 that the conformation codes (Stereo) are different in the place other than the place where the configuration codes (Configuration) are different, it is determined at ST8 that both have different configurations and conformations. With respect to a-Glc-4C, a-Gal-1C and a-Man-1C correspond thereto.

As described above, using conformation codes (Stereo) and configuration codes (Configuration), it is possible to very easily know the difference in stereochemistry between molecules.

Furthermore, it is also possible to easily determine whether molecules to be compared have the relationship between enantiomers (enantiomer), as follows. That is, with respect to both of conformation codes (Stereo) and configuration codes (Configuration), codes having the relationship obtained by reflecting a clock in a mirror are assigned to enantiomers (enantiomer). For example, the codes have the relationships between "on" and "el" and between "tw" and "te", and the codes of "ze" and "si" have the relationship where the codes do not vary. Therefore, "on" is converted into "el" and "tw" is converted into "te", and "ze" or "si" remains as it is, so that codes of one of the conformation codes (Stereo) and so forth are converted into enantiomorphic codes. Then, it is examined whether the converted and described conformation codes (Stereo) are coincident with conformation codes (Stereo) to be compared therewith, so that it is possible to determine whether molecules to be compared have the relationship between enantiomers (enantiomer).

The partial structure recognition is as follows. That is, while the above described example has related to recognition of the whole molecule, it is necessary to identify a partial conformation of a partial structure and to compare relative arrangements in order to apply the present invention to the prediction of the chemical shift of ¹³C-NMR. According to the present invention, this can be achieved by combining and comparing conformation codes (Stereo) and configuration codes (Configuration) starting from a node other than H spaced from a carbon atom, which is to be noticed, by three bonds if necessary.

With respect to application to ¹³C-NMR, when the chemical shift of ¹³C-NMR is predicted by utilizing an intelligent data base, conformation codes (Stereo) and configuration codes (Configuration) starting from a node other than H spaced from a carbon atom, which is to be noticed, by three bonds may be compared from a depth of level n of the node of the start atom to a depth of level n+3 or more thereof. The fact that the codes are coincident with each other at a deeper level corresponds to the fact that the codes have higher similarity. By comparing the codes up to the deepest level, it is possible to compare the whole molecule. If it is confirmed between molecules to be compared that the planar structure codes (CANOST) are coincident with each other and that the configuration codes (Configuration) and conformation codes (Stereo) are coincident with each other at a deeper level, it is possible to more precisely predict the chemical shift of ¹³C-NMR. As a result, by combining and utilizing planar structure codes (CANOST), configuration codes (Configuration) and conformation codes (Stereo), it is possible to precisely predict the chemical shift of ¹³C-NMR.

In addition, a program, in which a stereochemical coding method for taking stereochemistry about each of a plurality of atoms constituting a molecule to code the molecule has been described, can be recorded in a computer readable recording media.

As described above, according to the present invention, it is possible to computer-readably and canonically code information on the steric structural environment, such as conformation and configuration, of a molecule.

## Claims

1. A molecular stereochemical coding method for taking a stereochemistry about each of a plurality of atoms constituting a molecule to code the molecule, said method comprising:
a hierarchy classifying step of assigning a start atom, which is to be noticed, to a zero-th hierarchy serving as the lowest hierarchy, assigning an atom, which is combined with said start atom on a higher hierarchy side, to a first hierarchy, assigning an atom, which is combined with said atom assigned to said first hierarchy, to a second hierarchy, and similarly, sequentially assigning atoms to hierarchies until the final hierarchy which is set so as to be specially requested;
a molecular tree forming step of setting a predetermined precedence rule for placing a plurality of atoms, which belong to the same hierarchy, in the order, and placing said atoms, which belong the same hierarchy, in the order every hierarchy in accordance with said precedence rule, to form a molecular tree every said start atom from a lower hierarchy to a higher hierarchy so as to express a bonding relationship between said plurality of atoms;
a coding step of noticing one of atoms, which are assigned to the (n+3)-th hierarchy, with respect to each of integers n assuming that n is an integer of 0 or more, in said molecular tree, deriving a dihedral angle between a plane, which is formed by an atom in the (n+3)-th hierarchy, an atom in the (n+2)-th hierarchy and an atom in the (n+1)-th hierarchy, and a plane, which is formed by the atom in the (n+2)-th hierarchy, the atom in the (n+1)-th hierarchy and an atom in the n-th hierarchy, with respect to a group comprising four atoms which consists of the noticed atom in the (n+3)-th hierarchy, the atom in the (n+2)-th hierarchy which is combined with the atom in the (n+3)-th hierarchy, the atom in the (n+1)-th hierarchy which is combined with the atom in the (n+2)-th hierarchy, and the atom in the n-th hierarchy which is combined with the atom in the (n+1)-th hierarchy, replacing the derived dihedral angle into an angular symbol, which is defined in accordance with a predetermined angle dividing rule, in accordance with the magnitude of the dihedral angle, giving the replaced angular symbol to the noticed atom in the (n+3)-th hierarchy, and similarly, giving angular symbols in accordance with the magnitudes of the dihedral angles with respect to other atoms to be noticed; and
setting a predetermined linear notation rule for expressing said molecular tree by a row of characters, carrying out the linear notation of a set of said plurality of angular symbols in accordance with said predetermined linear notation rule so as to correspond to said molecular tree, preparing a conformation code indicative of a conformation of the molecule with respect to said start atom, and similarly, preparing conformation codes with respect to other start atoms.

2. A molecular stereochemical coding method as set forth in claim 1, which further comprises a configuration code preparing step of preparing a configuration code indicative of a configuration of the molecule for every said start atom, and wherein said configuration code preparing step causes an atom, which is to be noticed in the n-th hierarchy, to be positioned at a reference position in said angle dividing rule, integrally rotates all of said atoms belonging to the (n+3)-th hierarchy around a bonding axis, which connects the atom in the (n+1)-th hierarchy to the atom in the (n+2)-th hierarchy, so that an atom, which has a predetermined precedence in accordance with said precedence rule among said atoms belonging to the (n+3)-th hierarchy, is positioned at a predetermined angular position with respect to said reference position, gives an angular symbol according to said angle dividing rule to each of said atoms belonging to the (n+3)-th hierarchy, in accordance with an angular position after rotation with respect to said reference position, carries out the linear notation of a set of said plurality of angular symbols in accordance with said predetermined linear notation rule so as to correspond to said molecular tree, and prepares a configuration code for every said start atom.

3. A molecular stereochemical coding method as set forth in claim 1 or 2, which further comprises a planar structure code preparing step of preparing a planar structure code indicative of a planar structure of the molecule every said start atom, and wherein said planar structure code preparing step expresses said molecular tree by a set of planar structure symbols which planar-structurally express a bonding relationship between said plurality of atoms, carries out the linear notation of said set of planar structure symbols in accordance with said predetermined linear notation rule so as to correspond to said molecular tree, prepares a planar structure code indicative of the planar structure of the molecule with respect to said start atom, and similarly, prepares planar structure codes with respect to other start atoms.

4. A molecular stereochemical coding method as set forth in claim 3, wherein said conformation codes, said configuration codes and said planar structure codes are expressed in parallel with respect to said start atoms.

5. A molecular stereochemical coding method as set forth in claim 2, wherein when it is impossible to rotate said atoms belonging to the (n+3)-th hierarchy around the bonding axis connecting the atom belonging to the (n+1)-th hierarchy to the atom belonging to the (n+2)-th hierarchy, said angular symbols given at said coding step are adopted as they are, and the linear notation of a set of said plurality of angular symbols is carried out in accordance with said predetermined linear notation rule so as to correspond to said molecular tree, to prepare said configuration codes for every said start atom.

6. A molecular stereochemical coding method as set forth in claim 3, wherein said planar structural codes are CANOST linear notations.

7. A molecular stereochemical coding method as set forth in claim 1, wherein said precedence rule is a CANOST code precedence rule.

8. A molecular stereochemical coding method as set forth in claim 1, wherein said predetermined linear notation rule is a CANOST code linear notation rule.

9. A molecular stereochemical coding method as set forth in claim 1, wherein at said molecular tree forming step, a group of atoms having a low degree of notice in the identification of stereochemistry are replaced with predetermined symbols to be masked.

10. A molecular stereochemical coding method as set forth in claim 1, wherein said predetermined angle dividing rule divides an angle of 360 degrees into a predetermined number of clock-dial-like angular ranges, and the divided angular ranges are reflected in the level of abundance to be unequally divided.

11. A molecular stereochemical coding method as set forth in claim 1, wherein at said conformation code preparing step, said conformation codes are prepared with respect to at least two of said start atoms, the hierarchy numbers of which are spaced from each other by three hierarchies or more.

12. A computer readable recording media, in which a program for taking a stereochemistry about each of a plurality of atoms constituting a molecule to code the molecule has been recorded, said program comprising:
a hierarchy classifying step of assigning a start atom, which is to be noticed, to a zero-th hierarchy, assigning an atom, which is combined with said start atom on a higher hierarchy side, to a first hierarchy, assigning an atom, which is combined with said atom assigned to said first hierarchy, to a second hierarchy, and similarly, sequentially assigning atoms to hierarchies until the final hierarchy which is set so as to be specially requested;
a molecular tree forming step of setting a predetermined precedence rule for placing a plurality of atoms, which belong to the same hierarchy, in the order, and placing said atoms, which belong the same hierarchy, in the order every hierarchy in accordance with said precedence rule, to form a molecular tree every said start atom from a lower hierarchy to a higher hierarchy so as to express a bonding relationship between said plurality of atoms;
a coding step of noticing one of atoms, which are assigned to the (n+3)-th hierarchy, with respect to each of integers n assuming that n is an integer of 0 or more, in said molecular tree, deriving a dihedral angle between a plane, which is formed by an atom in the (n+3)-th hierarchy, an atom in the (n+2)-th hierarchy and an atom in the (n+1)-th hierarchy, and a plane, which is formed by the atom in the (n+2)-th hierarchy, the atom in the (n+1)-th hierarchy and an atom in the n-th hierarchy, with respect to a group comprising four atoms which consists of the noticed atom in the (n+3)-th hierarchy, the atom in the (n+2)-th hierarchy which is combined with the atom in the (n+3)-th hierarchy, the atom in the (n+1)-th hierarchy which is combined with the atom in the (n+2)-th hierarchy, and the atom in the n-th hierarchy which is combined with the atom in the (n+1)-th hierarchy, replacing the derived dihedral angle into an angular symbol, which is defined in accordance with a predetermined angle dividing rule, in accordance with the magnitude of the dihedral angle, giving the replaced angular symbol to the noticed atom in the (n+3)-th hierarchy, and similarly, giving angular symbols in accordance with the magnitudes of the dihedral angles with respect to other atoms to be noticed; and
setting a predetermined linear notation rule for expressing said molecular tree by a row of characters, carrying out the linear notation of a set of said plurality of angular symbols in accordance with said predetermined linear notation rule so as to correspond to said molecular tree, preparing a conformation code indicative of a conformation of the molecule with respect to said start atom, and similarly, preparing conformation codes with respect to other start atoms.
